# EUROPÄISCHE PATENTANMELDUNG

(11) **EP 4 212 212 A1**
(43) Veröffentlichungstag der Anmeldung: **19.07.2023**
(21) Anmeldenummer: 23151721.0
(22) Anmeldetag: 16.01.2023
(51) Int. Cl.: A62B 7/02, B63C 11/32, A61G 10/02, A61M 16/00

(54) **ATEMSTATION FÜR EINE DEKOMPRESSIONSKAMMER, ANORDNUNG MIT EINER DEKOMPRESSIONSKAMMER UND EINER ATEMSTATION UND VERFAHREN ZUM BETREIBEN EINER SOLCHEN DEKOMPRESSIONSKAMMER**

(30) Priorität: 17.01.2022 DE 102022100889
(71) Anmelder: Dräger Safety AG & Co. KGaA, 23560 Lübeck (DE)
(72) Erfinder: Neervoort, Sven, 23558 Lübeck (DE); Zimmermann, Tim, 23558 Lübeck (DE)
(74) Vertreter: Meyer-Gramann, Klaus Dieter

(57) **Zusammenfassung**

Die Erfindung betrifft eine mobile Atemstation (50) zur Verwendung in einer Dekompressionskammer (100) sowie eine Dekompressionskammer (100) mit einer solchen Atemstation (50) und ein Verfahren zum Betreiben einer solchen Dekompressionskammer (100). Eine personenseitige Koppeleinheit (8) der Atemstation (50) ist mit dem Atemsystem eines Menschen (P) verbunden. Eine Inspirations-Koppeleinheit (21) und eine Exspirations-Koppeleinheit (9) der Atemstation (50) lassen sich mit zwei korrespondierenden Koppeleinheiten (2.1 bis 2.4, 10.1 bis 10.4) einer Dekompressionskammer (100) lösbar verbinden. Die Atemstation (50) leitet ein Gasgemisch umfassend Sauerstoff von der Inspirations-Koppeleinheit (21) durch eine Inspirations-Fluidverbindung und eine Inspirations-Fluidführungseinheit (7) hindurch zu der personenseitigen Koppeleinheit (8). Ausgeatmete Luft fließt von der personenseitigen Koppeleinheit (8) durch eine Exspirations-Fluidführungseinheit (17) und eine Exspirations-Fluidverbindung hindurch zu der Exspirations-Koppeleinheit (9).

## Beschreibung

Die Erfindung betrifft eine mobile Atemstation zur Verwendung in einer Dekompressionskammer. Die Erfindung betrifft weiterhin eine Anordnung umfassend eine solche mobile Atemstation und eine Dekompressionskammer mit einer Koppelstation für eine solche mobile Atemstation sowie ein Verfahren zum Betreiben einer solchen Anordnung.

Eine Dekompressionskammer mit mindestens einer Atemstation wird dafür verwendet, um das Atemsystem sowie das gesamte durchblutete Gewebe eines Menschen zu dekomprimieren, nachdem dieser Mensch unter Überdruck gearbeitet hat. Beispielsweise hat dieser Mensch unter Wasser oder in einem Tunnel gearbeitet. Dieses Dekomprimieren ist erforderlich, um zu vermeiden, dass der Mensch schwere gesundheitliche Schäden erleidet, nachdem er nicht mehr dem Überdruck ausgesetzt ist. Diese gesundheitlichen Schäden sind unter den Bezeichnungen Taucherkrankheit, Dekompressionskrankheit oder Caisson-Krankheit bekannt. Randbedingungen für das Dekomprimieren eines Menschen werden in Deutschland durch die "Verordnung über Arbeiten in Druckluft (Druckluftverordnung)" geregelt. Ein Mensch in der Dekompressionskammer kann sich mit der oder einer Atemstation verbinden und dann einatmen und ausatmen. Der Druck in der Dekompressionskammer wird in einer gesteuerten Weise abgesenkt.

Im Folgenden wird abkürzend davon gesprochen, dass "der Mensch dekomprimiert wird". Damit ist gemeint, dessen Atemsystem und dessen durchblutetes Gewebe zu dekomprimieren.

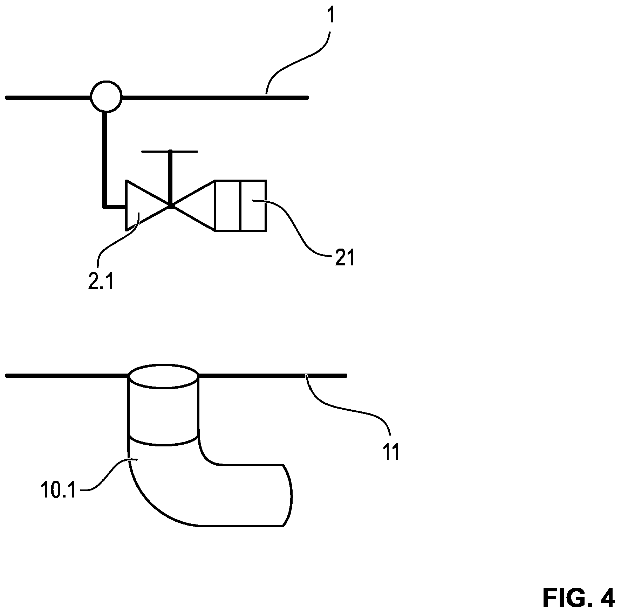

In DE 20 2005 014 078 U1 wird eine Anordnung mit einer Kompressionskammer 51, einer tragbaren Steuer- und Regelvorrichtung 10 in einem Koffer 11 und einer Unterdruckerzeugungsvorrichtung 31 mit einem Rahmen 30 beschrieben, vgl. Fig. 4. Die Steuer- und Regelvorrichtung 10 ist mit drei Zuführverbindungen 22 für frische Atemluft und drei Abführverbindungen 23 für ausgeatmete Luft verbunden. Eine Atemmaske lässt sich an eine Zuführverbindung 22 und an eine Abführverbindung 23 anschließen. Die Steuer- und Regelvorrichtung 10 lässt sich über zwei Schnellkupplungen 43 lösbar mit zwei Leitungen der Kompressionskammer 51 verbinden.

Der Erfindung liegt die Aufgabe zugrunde, eine Atemstation zur Verwendung in einer Dekompressionskammer bereitzustellen, wobei die Atemstation es ermöglicht, dass die Dekompressionskammer sich flexibler als bekannte Dekompressionskammern verwenden lässt. Weiterhin liegt der Erfindung die Aufgabe zugrunde, ein Verfahren zum Betreiben einer Anordnung mit einer solchen Atemstation bereitzustellen.

Die Aufgabe wird durch eine Atemstation mit den Merkmalen des Anspruchs 1 und durch ein Verfahren mit den Merkmalen des Anspruchs 15 gelöst. Vorteilhafte Ausgestaltungen sind in den Unteransprüchen angegeben. Vorteilhafte Ausgestaltungen der erfindungsgemäßen Atemstation sind, soweit sinnvoll, auch vorteilhafte Ausgestaltungen einer Anordnung mit mindestens einer erfindungsgemäßen Atemstation sowie des erfindungsgemäßen Verfahrens zum Betreiben einer solchen Anordnung.

Die erfindungsgemäße Atemstation ist mobil, d.h. sie lässt sich von einem Menschen tragen, bevorzugt ohne Hilfsmittel. Sie ist dazu ausgestaltet, in einer Dekompressionskammer verwendet zu werden. Diese Dekompressionskammer vermag einen Menschen zu dekomprimieren, während der Mensch sich im Inneren der Dekompressionskammer aufhält und mit der erfindungsgemäßen Atemstation verbunden ist.

Die erfindungsgemäße Atemstation umfasst
- eine personenseitige Koppeleinheit,
- eine Inspirations-Koppeleinheit und eine Exspirations-Koppeleinheit sowie
- eine Inspirations-Fluidführungseinheit und eine Exspirations-Fluidführungseinheit.

Unter einer "Fluidführungseinheit" wird ein Bauteil verstanden, welches ein Fluid entlang einer durch das Bauteil vorgegebenen Trajektorie zu führen vermag und bevorzugt verhindert, dass das Fluid seitlich oder schräg von der Trajektorie weg fließt. Ein flexibler und / oder biegsamer Schlauch und eine starre Röhre sind zwei Beispiele für eine Fluidführungseinheit. Unter einer "Koppeleinheit" wird ein Bauteil verstanden, welches sich lösbar mit einem anderen Bauteil, das als weitere Koppeleinheit fungiert, oder mit einem Menschen verbinden lässt. Nachdem diese Verbindung hergestellt ist, ist eine Fluidverbindung hergestellt. Im Falle von zwei Koppeleinheiten führt die Fluidverbindung durch die beiden Koppeleinheiten. Die Verbindung zwischen den beiden Bauteilen lässt sich wieder lösen.

Die personenseitige Koppeleinheit lässt sich auf das Gesicht eines Menschen aufbringen und ist dann ist wenigstens zeitweise mit dem Atemsystem dieses Menschen verbunden oder lässt sich mit seinem Atemsystem verbinden. Bevorzugt kann der Mensch nur durch die personenseitige Koppeleinheit hindurch einatmen und ausatmen. Die personenseitige Koppeleinheit ist sowohl mit der Inspirations-Fluidführungseinheit als auch mit der Exspirations-Fluidführungseinheit fluiddicht verbunden.

Die Inspirations-Koppeleinheit steht in einer Inspirations-Fluidverbindung mit der Inspirations-Fluidführungseinheit. Die Exspirations-Koppeleinheit steht in einer Exspirations-Fluidverbindung mit der Exspirations-Fluidführungseinheit.

Sowohl die Inspirations-Koppeleinheit als auch die Exspirations-Koppeleinheit der Atemstation lassen sich lösbar mit jeweils einer korrespondierenden Koppeleinheit der Dekompressionskammer fluiddicht verbinden oder sind mit der korrespondierenden Koppeleinheit verbunden. Diese beiden korrespondierenden Koppeleinheiten bilden eine Koppelstation der Dekompressionskammer. Diese beiden Verbindungen lassen sich wieder lösen. Nach Herstellung einer solchen lösbaren Verbindung ist eine Fluidverbindung zwischen der Koppeleinheit der Atemstation und der korrespondierenden Koppeleinheit der Dekompressionskammer hergestellt.

Wenn die beiden Verbindungen hergestellt und ein Mensch in der Dekompressionskammer mit der personenseitigen Koppeleinheit verbunden sind, so vermag die erfindungsgemäße Atemstation folgende beiden Zustände herzustellen und / oder folgende Schritte durchzuführen:
- Ein Gasgemisch umfassend Sauerstoff fließt von einer korrespondierenden Koppeleinheit der Dekompressionskammer zur Inspirations-Koppeleinheit und wird von dort durch die Inspirations-Fluidverbindung und die Inspirations-Fluidführungseinheit hindurch zur personenseitigen Koppeleinheit geleitet. Der verbundene Mensch kann dieses Gasgemisch einatmen.
- Ein Gasgemisch, das der verbundene Mensch in die personenseitige Koppeleinheit hinein ausgeatmet hat, wird von der personenseitigen Koppeleinheit durch die Exspirations-Fluidführungseinheit und die Exspirations-Fluidverbindung hindurch zur Exspirations-Koppeleinheit geleitet und fließt von dort zur korrespondierenden Koppeleinheit der Dekompressionskammer. Das ausgeatmete Gasgemisch kann aus der Dekompressionskammer abgeleitet werden.

Die Fluidführungseinheiten sind so ausgestaltet, dass sie bis zu einem gewissen Grad Druckunterschieden zwischen ihrem Inneren und ihrer Umgebung standhalten können. Bevorzugt sind die beiden Fluidführungseinheiten fluiddicht ausgelegt, in der Regel fluiddicht bis auf unvermeidliche Undichtigkeiten. Die beiden Fluidführungseinheiten können räumlich voneinander beabstandet sein. Möglich ist auch, dass die eine Fluidführungseinheit im Inneren der anderen Fluidführungseinheit angeordnet ist, beispielsweise nach Art eines Zwei-Lumen-Schlauchs.

Erfindungsgemäß ist die Atemstation mobil, d.h. ein Mensch vermag die Atemstation mit der personenseitigen Koppeleinheit zu tragen. Die Inspirations-Koppeleinheit und die Exspirations-Koppeleinheit der Atemstation lassen sich mit zwei korrespondierenden Koppeleinheiten einer Dekompressionskammer fluiddicht verbinden, und diese Verbindungen zwischen den beiden Koppeleinheiten der Atemstation und den korrespondierenden Koppeleinheiten der Dekompressionskammer lassen sich wieder lösen. Dadurch wird ermöglicht, dieselbe Atemstation nacheinander in unterschiedlichen Dekompressionskammern zu verwenden, wobei jede Dekompressionskammer jeweils mindestens ein Paar von korrespondierenden kammerseitigen Koppeleinheiten umfasst. Ein solches Paar wird nachfolgend auch als "Koppelstation" bezeichnet. Eine erfindungsgemäße Atemstation lässt sich lösbar mit einer Koppelstation verbinden. Möglich ist auch, dieselbe erfindungsgemäße Atemstation wahlweise an einer von mehreren möglichen Stellen in einer Dekompressionskammer zu verwenden, nämlich dann, wenn diese Dekompressionskammer mehrere Koppelstationen, also mehrere Paare von korrespondierenden Koppeleinheiten, umfasst, wobei diese Koppelstationen räumlich voneinander beabstandet sind. Die Atemstation lässt sich mit jeder dieser Koppelstationen lösbar verbinden, natürlich zu einem Zeitpunkt nur mit einer Koppelstation.

Die Erfindung ermöglicht es, eine Anordnung umfassend eine Dekompressionskammer mit m kammerseitigen Koppelstationen sowie n erfindungsgemäßen mobilen Atemstationen bereitzustellen, wobei n >= m > 1 ist. Eine Ausgestaltung der Erfindung betrifft eine derartige Anordnung. Zu jedem Zeitpunkt sind bis zu m Atemstationen mit jeweils einer korrespondierenden Koppelstation verbunden. Bevorzugt gilt n > m, und mindestens n - m Atemstationen werden in Reserve gehalten oder woanders verwendet oder überprüft. Falls eine verbundene Atemstation funktionsunfähig wird oder zu reinigen ist, so lässt sie sich rasch durch eine funktionsfähige und in Reserve gehaltene Atemstation ersetzen. Dadurch stehen - bis auf die kurze Zeitspanne, in der die defekte Atemstation durch die in Reserve gehaltene ersetzt wird - durchgehend m Atemstationen in der Dekompressionskammer zur Verfügung, um gleichzeitig bis zu m Personen gleichzeitig zu komprimieren. Dies wäre nicht möglich, wenn die Dekompressionskammer m festverbundene Atemstationen aufweisen würde. In diesem Falle müsste eine defekte festverbundene Atemstation in der Dekompressionskammer repariert werden und stünde während der Reparatur nicht zur Verfügung.

Die Reparatur einer Atemstation erfordert Zeit und in der Regel Werkzeuge. Falls mindestens eine Atemstation in der Dekompressionskammer in Reserve gehalten wird, so lässt sich in vielen Fällen in der Dekompressionskammer eine defekte Atemstation durch die in Reserve gehaltene Atemstation ersetzen, ohne dass hierfür ein Techniker von außen die Dekompressionskammer zu betreten braucht. Daher ist es in vielen Fällen auch nicht erforderlich, eine Tür zu öffnen oder den Druck in der Dekompressionskammer für den Austausch abzusenken. Die gerade beschriebene Ausgestaltung spart also Zeit und in der Regel elektrische Energie ein. Möglich ist auch, eine in Reserve gehaltene Atemstation in der Dekompressionskammer zu desinfizieren und / oder zu reinigen.

Bevorzugt umfasst die Atemstation genau eine personenseitige Koppeleinheit, z.B. eine Atemmaske, also nicht zwei oder mehr derartige Koppeleinheiten. Eine Ausgestaltung mit n erfindungsgemäßen Atemstationen, die jeweils genau eine personenseitige Koppeleinheit umfassen, ist flexibler als eine einzige Atemstation mit n Koppeleinheiten, weil sich jede der n Atemstationen unabhängig von jeder anderen der n Atemstationen verwenden und auch lösen und reparieren lässt.

In manchen Fällen lässt sich dank der lösbaren Verbindungen die Anzahl der erforderlichen Atemstationen reduzieren. An einem Einsatzort brauchen nur so viele Atemstationen vorrätig gehalten zu werden, wie Menschen gleichzeitig an diesem Einsatzort zu dekomprimieren sind, sowie optional Atemstationen, die in der Dekompressionskammer in Reserve vorrätig gehalten werden.

Außerdem ist es möglich, dass einem Menschen eine bestimmte erfindungsgemäße mobile Atemstation dauerhaft oder wenigstens für mehrere Einsätze zugeordnet ist. Diese mobile Atemstation kann an respiratorische und sonstige Eigenschaften dieses Menschen angepasst sein. Außerdem sind die Anforderungen an ein Desinfizieren und an sonstige hygienische Anforderungen in manchen Fällen geringer, wenn einem Menschen dauerhaft dieselbe Atemstation zugeordnet ist, als wenn dieselbe Atemstation nacheinander von unterschiedlichen Menschen verwendet werden würde. Der Mensch kann die ihm zugeordnete Atemstation nacheinander mit unterschiedlichen Koppelstationen verbinden und die Verbindung wieder lösen. Selbstverständlich können mehreren Menschen jeweils eine erfindungsgemäße Atemstation zugeordnet sein.

Erfindungsgemäß lassen sich die beiden fluiddichten Verbindungen zwischen den beiden Koppeleinheiten der Atemstation und den beiden korrespondierenden Koppeleinheiten der Dekompressionskammer herstellen und wieder lösen. Bevorzugt lassen sich diese beiden Verbindungen ohne Verwendung eines Werkzeugs herstellen und wieder lösen, beispielsweise mithilfe von Schnellkupplungen. Daher kann ein Mensch, der eine erfindungsgemäße Atemstation mit sich führt, diese Atemstation mit den beiden korrespondierenden Koppeleinheiten der Dekompressionskammer verbinden und wieder lösen, ohne hierfür ein Werkzeug mit sich führen zu müssen. Daher kann in vielen Fällen der zu dekomprimierende Mensch selber die Atemstation verbinden, also an eine Koppelstation anschließen, und wieder lösen. Möglich, aber dank der Erfindung nicht erforderlich ist, dass ein Techniker die Dekompressionskammer betritt, um die Verbindungen herzustellen und wieder zu lösen.

In einer bevorzugten Ausgestaltung umfasst die erfindungsgemäße Atemstation einen Inspirations-Lungenautomaten. Ein Eingang dieses Inspirations-Lungenautomaten steht in einer Fluidverbindung mit der Inspirations-Koppeleinheit, ein Ausgang in einer Fluidverbindung mit der Inspirations-Fluidführungseinheit. Diese beiden Fluidverbindungen gehören zu der Inspirations-Fluidverbindung, welche die Atemstation herstellt. Die Inspirations-Fluidverbindung der Atemstation führt weiterhin durch den Inspirations-Lungenautomaten. Der Inspirations-Lungenautomat vermag den Druck, der an der personenseitigen Koppeleinheit anliegt, mit folgendem Ziel zu verändern: Der anliegende Druck folgt einem Druck in der Umgebung der Atemstation, insbesondere einem Druck im Inneren einer Dekompressionskammer, in der die Atemstation verwendet wird. Diese Ausgestaltung erleichtert es, den Menschen gemäß eines vorgegebenen zeitlichen Druckverlaufs zu dekomprimieren. Dieser Druckverlauf wird im Inneren der Dekompressionskammer hergestellt. Insbesondere dank des Inspirations-Lungenautomaten kann dieses Dekomprimieren automatisch ablaufen. Ausreichend ist, den Druck im Inneren der Dekompressionskammer gesteuert zu verändern. Der Druck an jeder personenseitigen Koppeleinheit, die in der Dekompressionskammer verwendet wird, folgt diesem Druck in der Dekompressionskammer.

Bevorzugt ist der Druck am Ausgang des Inspirations-Lungenautomaten stets kleiner als oder höchstens gleich dem Druck am Eingang. Dadurch ist der Druck an der personenseitigen Koppeleinheit auch kleiner als oder höchstens genauso groß wie der Druck in der Inspirations-Koppeleinheit. Dadurch ist es nicht erforderlich, den Druck in der Inspirations-Koppeleinheit oder in einer Fluidführungseinheit im Inneren der Dekompressionskammer, die zur Inspirations-Koppeleinheit führt, zu regeln oder zu steuern. In vielen Fällen ist es außerdem nicht erforderlich, den Druck in einer stationären Fluidführungseinheit der Dekompressionskammer zu steuern oder zu regeln, wobei diese stationäre Fluid Führungseinheit zu den Koppelstationen der Dekompressionskammer führt. Ausreichend ist in vielen Fällen, den Druck in der Umgebung der Atemstation zu regeln oder zu steuern.

Falls in der Dekompressionskammer mehrere Atemstationen verwendet werden, umfasst bevorzugt jede verwendete Atemstation jeweils einen solchen Inspirations-Lungenautomaten.

In einer bevorzugten Realisierungsform umfasst der Inspirations-Lungenautomat drei Kammern, nämlich eine Vordruck-Kammer, eine Hinterdruck-Kammer und eine Steuerdruck-Kammer. Die Vordruck-Kammer steht in einer Fluidverbindung mit der Inspirations-Koppeleinheit und dadurch in einer Fluidverbindung mit einer korrespondierenden Fluidführungseinheit der Dekompressionskammer. Die Hinterdruck-Kammer steht in einer Fluidverbindung mit der Inspirations-Fluidführungseinheit und dadurch in einer Fluidverbindung mit der personenseitigen Koppeleinheit. Die Steuerdruck-Kammer steht in einer Fluidverbindung mit der Umgebung der Atemstation, so dass der Druck in der Steuerdruck-Kammer dem Druck in der Umgebung folgt. Die Vordruck-Kammer ist von der Steuerdruck-Kammer getrennt, und zwar bevorzugt durch eine flexible oder bewegliche Wand. Zwischen der Vordruck-Kammer und der Hinterdruck-Kammer besteht eine Fluidverbindung, wobei ein Verschluss diese Fluidverbindung wahlweise freizugeben oder zu versperren vermag. Der Verschluss gibt diese Fluidverbindung dann frei, wenn der Druck in der Steuerdruck-Kammer größer als der Druck in der Hinterdruck-Kammer ist. Bevorzugt gibt dieser Verschluss die Fluidverbindung nur dann frei, wenn der Druck in der Steuerdruck-Kammer größer als der Druck in der Hinterdruck-Kammer ist.

Beispielsweise steht dieser Verschluss in einer mechanischen Wirkverbindung mit der gerade beschriebenen flexiblen oder beweglichen Wand. Bevorzugt folgt der Druck in der Vordruck-Kammer dem Druck in der Steuerdruck-Kammer.

Die Realisierungsform ermöglicht es, den Inspirations-Lungenautomaten als rein mechanisches Bauteil auszugestalten, also ohne dass der Inspirations-Lungenautomat einen elektrischen Antrieb oder ein Steuergerät aufweist. Daher ist es oft nicht erforderlich, den Inspirations-Lungenautomaten mit elektrischer Energie zu versorgen.

In einer bevorzugten Ausgestaltung umfasst die erfindungsgemäße Atemstation weiterhin einen Exspirations-Lungenautomaten. Dieser Exspirations-Lungenautomat steht einerseits in einer Fluidverbindung mit der Exspirations-Fluidführungseinheit und damit mit der personenseitigen Koppeleinheit. Andererseits steht der Exspirations-Lungenautomat in einer Fluidverbindung mit der Exspirations-Koppeleinheit. Diese beiden Fluidverbindungen gehören zur Exspirations-Fluidverbindung. Die Exspirations-Fluidverbindung der Atemstation führt weiterhin durch den Exspirations-Lungenautomaten hindurch. Der Exspirations-Lungenautomat vermag eine Fluidverbindung zwischen der Exspirations-Fluidführungseinheit und der Exspirations-Koppeleinheit wahlweise herzustellen oder zu versperren. Der Exspirations-Lungenautomat stellt diese Fluidverbindung her oder gibt sie frei, wenn der Druck in der Exspirations-Fluidführungseinheit größer ist als der Druck in der Fluidverbindung zur Exspirations-Koppeleinheit, und versperrt ansonsten diese Fluidverbindung. Bei hergestellter Fluidverbindung wird ein Druckausgleich zwischen der Exspirations-Fluidführungseinheit und der Exspirations-Koppeleinheit ermöglicht.

In vielen Fällen bewirkt der Exspirations-Lungenautomat, dass der Druck in der Exspirations-Fluidführungseinheit stets größer als oder genauso groß wie der Druck in der Exspirations-Koppeleinheit ist, vorausgesetzt der verbundene Mensch atmet mit ausreichend großem Druck aus.

Der Exspirations-Lungenautomat verhindert, dass der Druck in der personenseitigen Koppeleinheit zu schnell abfällt und insbesondere unter den Druck in der Umgebung der Atemstation fällt. Der Exspirations-Lungenautomat reduziert dadurch die Gefahr, dass ein zu dekomprimierender Mensch zu rasch dekomprimiert wird. Außerdem ermöglicht es der Exspirations-Lungenautomat in vielen Fällen, den Menschen gemäß eines vorgegebenen zeitlichen Druckverlaufs zu dekomprimieren.

Falls in der Dekompressionskammer mehrere Atemstationen verwendet werden, umfasst bevorzugt jede verwendete Atemstation jeweils einen solchen Exspirations-Lungenautomaten.

In einer bevorzugten Realisierungsform umfasst der Exspirations-Lungenautomat drei Kammern, nämlich eine Vordruck-Kammer, eine Hinterdruck-Kammer und eine Steuerdruck-Kammer. Die Vordruck-Kammer steht in einer Fluidverbindung mit der Exspirations-Fluidführungseinheit und dadurch in einer Fluidverbindung mit der personenseitigen Koppeleinheit. Die Hinterdruck-Kammer steht in einer Fluidverbindung mit der Exspirations-Koppeleinheit und dadurch in einer Fluidverbindung mit einer korrespondierenden Koppeleinheit der Dekompressionskammer. Die Steuerdruck-Kammer steht in einer Fluidverbindung mit der Umgebung der Atemstation. Zwischen der Vordruck-Kammer und der Hinterdruck-Kammer besteht eine Fluidverbindung, wobei ein Verschluss diese Fluidverbindung wahlweise freizugeben oder zu versperren vermag. Der Verschluss gibt diese Fluidverbindung dann und nur dann frei, wenn der Druck in der Steuerdruck-Kammer kleiner als der Druck in der Vordruck-Kammer ist.

Bevorzugt folgt dank der Fluidverbindung der Druck in der Steuerdruck-Kammer dem Druck in der Umgebung der Atemstation. Die Vordruck-Kammer ist von der Steuerdruck-Kammer getrennt, und zwar bevorzugt durch eine flexible oder bewegliche Wand. Dadurch folgt der Druck in der Vordruck-Kammer dem Druck in der Steuerdruck-Kammer. Beispielsweise steht dieser Verschluss in einer mechanischen Wirkverbindung mit der gerade beschriebenen flexiblen oder beweglichen Wand.

Die gerade beschriebene Realisierungsform des Exspirations-Lungenautomaten mit den drei Kammern reduziert weiter das Risiko, dass der Druck in der Vordruck-Kammer zu rasch abfällt und dadurch die Person, die mit der personenseitigen Koppeleinheit verbunden ist, zu rasch dekomprimiert wird. Die Realisierungsform ermöglicht es, den Exspirations-Lungenautomaten als rein mechanisches Bauteil auszugestalten, also ohne dass der Exspirations-Lungenautomat einen elektrischen Antrieb oder ein Steuergerät aufweist. Daher ist es oft nicht erforderlich, den Exspirations-Lungenautomaten mit elektrischer Energie zu versorgen.

Besonders bevorzugt umfasst die Atemstation sowohl einen Inspirations-Lungenautomaten als auch einen Exspirations-Lungenautomaten, wobei beide Lungenautomaten bevorzugt so wie gerade beschrieben ausgestaltet sind. Falls ein Mensch mit der personenseitigen Koppeleinheit verbunden ist, so vermag die Atemstation dank der beiden Lungenautomaten bevorzugt automatisch folgende beiden Zustände herzustellen:
- Wenn der Mensch einatmet, gibt der Inspirations-Lungenautomat die Inspirations-Fluidverbindung frei und frei und bewirkt, dass der an der personenseitigen Koppeleinheit anliegende Druck einen Druck in der Umgebung der Atemstation folgt. Der Exspirations-Lungenautomat sperrt die Exspirations-Fluidverbindung.
- Wenn der Mensch ausatmet, gibt der Exspirations-Lungenautomat die Exspirations-Fluidverbindung frei. Bevorzugt gibt der Exspirations-Lungenautomat die Exspirations-Fluidverbindung nur dann frei, wenn der Druck in der Exspirations-Fluidführungseinheit größer als der Druck in der Umgebung der Atemstation ist. Der Inspirations-Lungenautomat sperrt die Inspirations-Fluidverbindung.

Diese Ausgestaltung mit den beiden Zuständen erhöht die Zuverlässigkeit, dass das Dekomprimieren des verbundenen Menschen einem vorgegebenen Ablauf folgt, insbesondere ein vorgegebener zeitlicher Druckverlauf an der personenseitigen Koppeleinheit eingehalten wird.

Bevorzugt arbeiten die beiden Lungenautomaten rein mechanisch und abhängig von Druckunterschieden. Insbesondere vermögen die beiden Lungenautomaten die gerade beschriebenen beiden Zustände mechanisch herzustellen. Diese rein mechanische Ausgestaltung erspart die Notwendigkeit, die Atemstation mit einem Stellglied und einem signalverarbeitenden Steuergerät zu versehen, wobei dieses Steuergerät die beiden Lungenautomaten ansteuert. Weiterhin ist es nicht erforderlich, eine Datenverbindung zwischen der Atemstation und der Dekompressionskammer herzustellen. Außerdem braucht die Atemstation nicht notwendigerweise eine Spannungsversorgungseinheit. Die erfindungsgemäße Atemstation lässt sich aber in Verbindung mit einem Steuergerät und / oder einer eigenen Spannungsversorgungseinheit verwenden.

Bevorzugt umfasst die erfindungsgemäße Atemstation ein Gehäuse, besonders bevorzugt ein starres und druckfestes Gehäuse. An diesem Gehäuse sind außen sowohl die Inspirations-Koppeleinheit als auch die Exspirations-Koppeleinheit befestigt, optional mithilfe jeweils einer Fluidführungseinheit. Besonders bevorzugt ist die personenseitige Koppeleinheit außerhalb des Gehäuses angeordnet, sodass das Gehäuse nicht geöffnet zu werden braucht, um die personenseitige Koppeleinheit zu verwenden. Die beiden Fluidführungseinheiten der Atemstation verbinden dieses Gehäuse fluiddicht mit der personenseitigen Koppeleinheit und sind bevorzugt als flexible Schläuche ausgestaltet. Bevorzugt ist mindestens einer der beiden gerade erwähnten optionalen Lungenautomaten in diesem Gehäuse untergebracht. Besonders bevorzugt sind beide Lungenautomaten in diesem Gehäuse untergebracht. Die Ausgestaltung mit dem Gehäuse führt einerseits zu einer besonders robusten mechanischen Ausgestaltung und schützt bis zu einem gewissen Grade die optionalen Lungenautomaten vor Beschädigungen von außen. Andererseits ist es möglich, die beiden Fluidführungseinheiten und die personenseitige Koppeleinheit außerhalb des Gehäuses anzubringen, sodass ein Mensch, der die Atemstation benutzen und sich mit der personenseitigen Koppeleinheit verbinden will, nicht das Gehäuse zu öffnen braucht.

Die Erfindung betrifft weiterhin eine Anordnung umfassend mindestens eine erfindungsgemäße Atemstation und eine Dekompressionskammer zum Dekomprimieren eines Menschen. Diese Ausgestaltung der Erfindung löst die Aufgabe, eine Anordnung mit einer Dekompressionskammer und mindestens einer Atemstation bereitzustellen, wobei die Anordnung sich flexibler verwenden lässt als bekannte derartige Anordnungen.

Bevorzugt ist diese Dekompressionskammer stationär, kann also von einem Menschen nur mit geeigneten Transportwerkzeug bewegt werden. Im Inneren dieser Dekompressionskammer wird eine Druckkammer gebildet. Im Inneren der Druckkammer lässt sich ein Überdruck relativ zur Umgebung erzeugen. Bevorzugt kann ein Mensch durch eine Tür in das Innere der Druckkammer gehen und dann die Tür verschließen, sodass der Überdruck erzeugt und aufrecht erhalten werden kann.

Die Anordnung umfasst weiterhin eine erfindungsgemäße Atemstation als eine erste Atemstation. Bevorzugte Ausgestaltungen der erfindungsgemäßen Atemstation sind auch bevorzugte Ausgestaltungen der Anordnung mit der Dekompressionskammer und der ersten Atemstation. Die Dekompressionskammer umfasst eine erste Koppelstation. Die erste Atemstation ist wenigstens zeitweise sind im Inneren der Druckkammer angeordnet, die erste Koppelstation dauerhaft. Möglich ist, dass die erste Atemstation zeitweise außerhalb der Druckkammer angeordnet ist, beispielsweise in Reserve gehalten oder gereinigt oder desinfiziert oder repariert wird.

Die erste Koppelstation umfasst eine Inspirations-Koppeleinheit und eine Exspirations-Koppeleinheit. Die Inspirations-Koppeleinheit der ersten Atemstation ist wenigstens zeitweise mit der Inspirations-Koppeleinheit der ersten Koppelstation lösbar verbunden oder lässt sich mit dieser lösbar verbinden. Die Exspirations-Koppeleinheit der ersten Atemstation ist wenigstens zeitweise mit der Exspirations-Koppeleinheit der ersten Koppelstation lösbar verbunden oder lässt sich mit dieser lösbar verbinden. Bevorzugt lassen diese beiden Verbindungen sich ohne Verwendung eines Werkzeugs herstellen und wieder lösen, beispielsweise mit jeweils einer Schnellkupplung.

Die Dekompressionskammer umfasst weiterhin eine kammerseitige Inspirations-Fluidführungseinheit und eine kammerseitige Exspirations-Fluidführungseinheit. Diese beiden kammerseitigen Fluidführungseinheiten sind bevorzugt mit der Druckkammer mechanisch verbunden. Optional führen die beiden kammerseitigen Fluidführungseinheiten - oder mindestens eine kammerseitige Fluidführungseinheit - durch eine Wandung der Druckkammer hindurch. Bevorzugt sind beide kammerseitigen Fluidführungseinheiten als starren Röhren ausgestaltet, können aber auch als Schläuche ausgestaltet sein.

Falls die erste Atemstation mit der ersten Koppelstation verbunden ist, so ist folgender Zustand hergestellt: Die Inspirations-Koppeleinheit der ersten Koppelstation steht in einer Fluidverbindung mit der kammerseitigen Inspirations-Fluidführungseinheit. Die Exspirations-Koppeleinheit der ersten Koppelstation steht in einer Fluidverbindung mit der kammerseitigen Exspirations-Fluidführungseinheit.

Falls die beiden lösbaren Verbindungen zwischen der ersten Atemstation und der Koppelstation hergestellt sind und ein Mensch mit der personenseitigen Koppeleinheit verbunden ist, so vermag die Dekompressionskammer folgende beiden Schritte durchzuführen:
- Ein Gasgemisch umfassend Sauerstoff wird von einer Quelle durch die kammerseitige Inspirations-Fluidführungseinheit, die erste Koppelstation und die erste Atemstation hindurch zur personenseitigen Koppeleinheit geleitet. Die Quelle kann innerhalb oder außerhalb der Druckkammer angeordnet sein. Der verbundene Mensch kann dieses Gasgemisch einatmen.
- Ein Gasgemisch, welches der verbundene Mensch ausgeatmet hat, wird von der personenseitigen Koppeleinheit durch die erste Atemstation, die erste Koppelstation und die kammerseitige Exspirations-Fluidführungseinheit hindurch aus der Druckkammer abgeführt.

Weiterhin umfasst die Dekompressionskammer eine Druckveränderungseinheit. Diese Druckveränderungseinheit vermag den Druck im Inneren der Druckkammer dergestalt zu verändern, dass der tatsächliche Druck einem vorgegebenen zeitlichen Verlauf des Solldrucks folgt. Bevorzugt legt dieser zeitliche Verlauf fest, dass der Druck kontinuierlich oder stufenweise abnimmt.

In einer Ausgestaltung umfasst die Druckveränderungseinheit einen Drucksensor, der ein Maß für den Druck im Inneren der Druckkammer misst, sowie ein signalverarbeitendes Steuergerät (Controller, Control Unit). Das Steuergerät vermag Messwerte von dem Drucksensor zu empfangen und abhängig von Messwerten des Drucksensors den Druck im Inneren der Druckkammer zu verändern. Das Steuergerät führt eine Regelung (Closed-loop Control) mit dem Regelungsziel durch, dass der tatsächliche Druck im Inneren der Druckkammer dem vorgegebenen zeitlichen Verlauf folgt. Das Steuergerät kann auch eine Steuerung (Open-loop Control) durchführen. Diese Steuerung erfordert nicht notwendigerweise einen Drucksensor.

Dieses Merkmal mit der Druckveränderungseinheit ermöglicht es, dass ein Mensch in der Druckkammer gemäß einem vorgegebenen zeitlichen Verlauf dekomprimiert wird. Ermöglicht wird, dass der Druck, der an der personenseitigen Koppeleinheit anliegt, dem geregelten oder gesteuerten Druck im Inneren der Druckkammer folgt.

Die gerade beschriebene erfindungsgemäße Anordnung umfasst eine Dekompressionskammer mit einer ersten Koppelstation sowie eine erste mobile Atemstation. In einer bevorzugten Ausgestaltung umfasst die Dekompressionskammer zusätzlich mindestens eine weitere Koppelstation, die von der ersten Koppelstation räumlich beabstandet ist. Die erfindungsgemäße erste Atemstation lässt sich wahlweise mit der ersten Koppelstation oder mit der oder einer weiteren Koppelstation lösbar verbinden.

In einer bevorzugten Ausgestaltung umfasst die Anordnung die Dekompressionskammer, die erste mobile Atemstation und zusätzlich eine zweite erfindungsgemäße mobile Atemstation. Mit der ersten Koppelstation lassen sich wahlweise die erste Atemstation oder die zweite Atemstation lösbar verbinden. Auch mit der optionalen zweiten Koppelstation lassen sich wahlweise die erste Atemstation oder die zweite Atemstation lösbar verbinden. Auch die zweite Atemstation befindet sich bevorzugt wenigstens zeitweise im Inneren der Druckkammer und kann sich zeitweise außerhalb der Druckkammer befinden.

Diese beiden Ausgestaltungen erhöhen weiter die Flexibilität der Anordnung mit der Dekompressionskammer.

In einer Realisierungsform umfasst die Anordnung eine Kombination der gerade beschriebenen Ausgestaltungen. Die Anordnung umfasst eine Dekompressionskammer mit m Koppelstationen und weiterhin n mobilen Atemstationen. Hierbei sind m und n Anzahlen, also zwei natürliche Zahlen, wobei n größer als oder gleich m und m größer als 1 ist. Mit anderen Worten: Die Anordnung umfasst mindestens zwei, bevorzugt mindestens drei mobile Atemstationen, und die Dekompressionskammer umfasst mindestens zwei Koppelstationen, wobei die Anzahl n der mobilen Atemstationen größer als oder genauso groß wie die Anzahl m der Koppelstationen in der Druckkammer ist. In einer Ausgestaltung stehen dadurch n - m >= 1 mobile Atemstationen, also mindestens eine mobile Atemstation, als Reserve zur Verfügung. Ein erfindungsgemäßes Verfahren spezifiziert ein Verfahren zum Betreiben einer solchen Anordnung.

Jede der n Atemstationen ist erfindungsgemäß ausgestaltet und lässt sich mit jeder der m Koppelstationen lösbar verbinden, natürlich nur jeweils eine Atemstation mit einer Koppelstation. Jede Koppelstation umfasst jeweils eine kammerseitige Inspirations-Koppeleinheit und eine kammerseitige Exspirations-Koppeleinheit. Falls die lösbaren Verbindungen hergestellt sind Komma so wird folgender Zustand bewirkt: Die Inspirations-Koppeleinheiten stehen in jeweils einer Fluidverbindung mit der kammerseitigen Inspirations-Fluidführungseinheit, die kammerseitigen Exspirations-Koppeleinheiten in jeweils einer Fluidverbindung mit der kammerseitigen Exspirations-Fluidführungseinheit. Die kammerseitigen Inspirations-Fluidführungseinheit steht in einer Fluidverbindung mit einer Quelle für das Gasgemisch, die kammerseitige Exspirations-Fluidführungseinheit in einer Fluidverbindung mit der Umgebung oder mit einer Fluidaufnahme.

Jede der n Atemstationen weist jeweils alle Merkmale von Anspruch 1 auf. In einer Ausgestaltung ist mindestens eine Atemstation gemäß mindestens einer der vorteilhaften Ausgestaltungen ausgestaltet, also gemäß mindestens einem der abhängigen Ansprüche 2 bis 9. Möglich ist, dass eine erste der n Atemstationen gemäß mindestens einer vorteilhaften Ausgestaltung ausgestaltet ist und eine zweite der n Atemstationen gemäß mindestens einer anderen vorteilhaften Ausgestaltung.

Eine derartig ausgestaltete Anordnung ermöglicht folgenden Modus: Bis zu m erfindungsgemäße Atemstationen werden gleichzeitig im Inneren der Druckkammer verwendet und sind gleichzeitig mit jeweils einer der m Koppelstationen der Dekompressionskammer sowie mit jeweils einem Menschen verbunden, wobei dieser Mensch dekomprimiert wird. Dadurch lassen sich bis zu m Menschen gleichzeitig dekomprimieren. Möglich ist, dass jede dieser m Atemstationen jeweils einem Menschen zugeordnet ist und nur von diesem Menschen verwendet wird, aber nicht von einem anderen Menschen. Dies reduziert den Aufwand, der zum Desinfizieren dieser m Atemstationen erforderlich ist.

Bevorzugt gilt n > m, so dass es mehr Atemstationen als Koppelstationen gibt. Die aktuell nicht verwendeten Atemstationen, also n - m >= 1 Atemstationen, werden in Reserve gehalten, und zwar bevorzugt im Inneren der Druckkammer. Die n - m in Reserve gehaltenen Atemstationen sind aktuell weder mit einer Koppelstation und noch mit einem Menschen verbunden. Falls eine der bis zu m Atemstationen, die verwendet werden oder verwendet werden sollen, sich als defekt erweist oder beim Einsatz defekt wird oder zu desinfizieren oder zu reinigen ist, so lässt sich diese defekte oder zu reinigende Atemstation durch eine der n -m Atemstationen, die in Reserve gehalten werden, ersetzen.

Bevorzugt werden die n - m Atemstationen im Inneren der Druckkammer in Reserve vorrätig gehalten. Falls diese in Reserve gehaltenen n - m Atemstationen sich bereits im Inneren der Druckkammer befinden, so ist es nicht erforderlich, eine Tür der Druckkammer zu öffnen, um von außen eine neue Atemstation zu besorgen. Ein Öffnen der Tür führt zwangsläufig zu einem Druckausgleich zwischen dem Inneren der Druckkammer und der Umgebung, und daher ist dieser Druckausgleich häufig nicht erwünscht. Falls der Defekt an der Atemstation auftritt, während diese Atemstation mit einem zu dekomprimierenden Menschen verbunden ist, so wird das Dekomprimieren dieses Menschen nur für die kurze Zeitspanne unterbrochen, in der die defekte Atemstation von dem Menschen und von der Koppelstation getrennt wird und eine bislang in Reserve gehaltene Atemstationen mit den Menschen und der Koppelstation verbunden wird. Oft ist es nicht erforderlich, eigens für diesen Austausch den Druck in der Druckkammer zu verändern.

Möglich ist auch, dass mindestens eine der n - m in Reserve gehaltenen Atemstationen sich außerhalb der Druckkammer befindet, beispielsweise für eine Überprüfung oder eine Reparatur oder Reinigung. Möglich ist, diese Atemstation dann wieder in das Innere der Druckkammer zu verbringen, insbesondere dann, wenn aktuell in dieser Druckkammer kein Mensch dekomprimiert wird.

Im Folgenden wird die Erfindung anhand eines Ausführungsbeispiels beschrieben. Hierbei zeigt
- Figur 1: schematisch eine Dekompressionskammer mit vier Koppelstationen und einer erfindungsgemäßen Atemstation;
- Figur 2: schematisch eine erfindungsgemäße Atemstation;
- Figur 3: in einer Seitenansicht die Atemstation von Figur 2;
- Figur 4: schematisch die beiden lösbaren Verbindungen zwischen einer Atemstation und einer Koppelstation;
- Figur 5: schematisch, wie eine Atemstation innen an der Druckkammer lösbar befestigt ist;
- Figur 6: einen Schnitt durch den Exspirations-Lungenautomaten;
- Figur 7: einen weiteren Schnitt durch den Exspirations-Lungenautomaten;
- Figur 8: eine perspektivische Darstellung des Exspirations-Lungenautomaten, wobei die Membrane fortgelassen ist;
- Figur 9: den Inspirations-Lungenautomaten in einer Detailansicht;
- Figur 10: den Inspirations-Lungenautomaten mit dem Ventil in einer sperrenden Position (links) und in einer freigebenden Position (rechts).

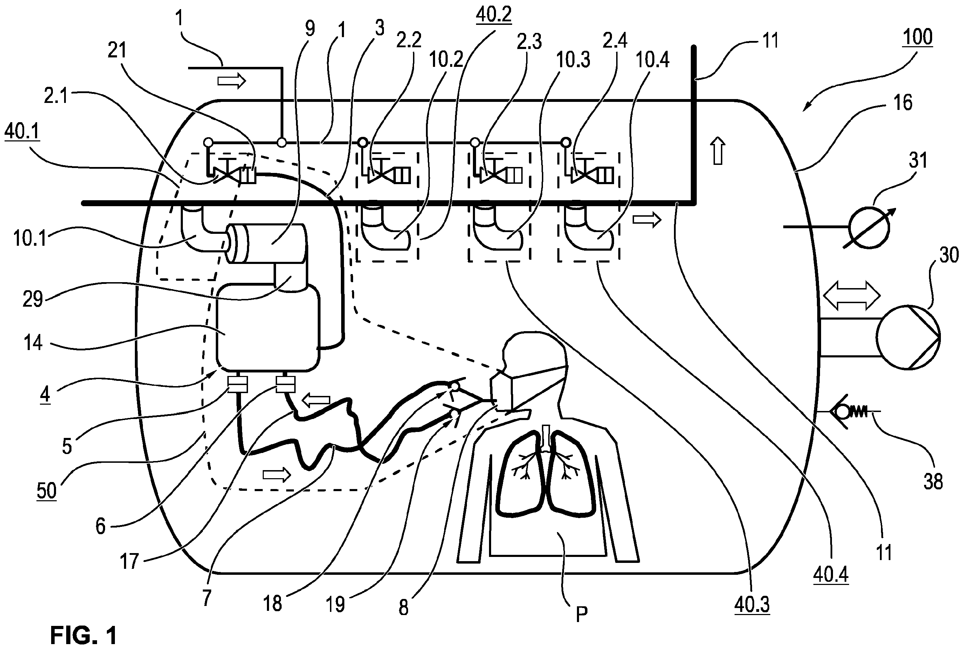

Figur 1 zeigt eine Dekompressionskammer 100, die beispielsweise ein Bestandteil einer Tunnelbohrmaschine ist, sowie schematisch einen Menschen P in dieser Dekompressionskammer 100. Die Dekompressionskammer 100 umfasst eine starre, druckfeste Druckkammer 16. Ein Bauteil mit einem Gehäuse ist druckfest, wenn das Gehäuse einem Unterschied zwischen einem Druck im Inneren und einem Druck außerhalb des Gehäuses bis zu einer vorgegebenen Schranke standhalten kann. Ein Mensch P kann die Druckkammer 16 durch eine nicht gezeigte Tür betreten, und die Tür lässt sich fluiddicht und druckdicht verschließen.

Im Inneren der Druckkammer 16 sind folgende Bestandteile fest montiert:
- eine Inspirations-Leitung 1, die druckfest und bevorzugt starr ist und die von einer Quelle außerhalb der Dekompressionskammer 100 durch eine Durchführung in der Druckkammer 16 in das Innere der Druckkammer 16 führt,
- eine Exspirations-Leitung 11, die ebenfalls Druckfest und bevorzugt starr ist und die durch eine Durchführung in der Druckkammer 16 aus der Dekompressionskammer 100 heraus in die Umgebung der Dekompressionskammer 100 führt, und
- vier Koppelstationen 40.1 bis 40.4 für jeweils eine erfindungsgemäße Atemstation.

Eine ansteuerbare Fluidfördereinheit 30, beispielsweise eine Pumpe, steht in einer Fluidverbindung mit dem Inneren der Dekompressionskammer 100 und kann den Druck in der Druckkammer 16 vergrößern. Ein ansteuerbares Ventil 38 lässt sich wahlweise schließen oder öffnen. Das geöffnete Ventil 38 reduziert einen Überdruck in der Druckkammer 16, weil eine Fluidverbindung zwischen dem Inneren der Druckkammer 16 und der Umgebung hergestellt ist und daher Gas aus der Druckkammer 16 abfließen kann. Bei geschlossenem Ventil ist diese Fluidverbindung unterbrochen, und ein Überdruck wird nicht abgebaut. Ein nicht gezeigtes signalverarbeitendes Steuergerät (Controller) vermag die Fluidfördereinheit 30 und das Ventil 38 anzusteuern und dadurch den Druck im Inneren der Druckkammer 16 einzustellen und bei Bedarf zu verändern.

In einer Ausgestaltung regelt das Steuergerät den Druck im Inneren der Druckkammer 16, sodass der tatsächliche Druck einem vorgegebenen zeitlichen Verlauf des Solldrucks folgt. Das Steuergerät hat Lesezugriff auf einen Datenspeicher, in dem in einer rechnerauswertbaren Form der vorgegebene zeitliche Verlauf des Solldrucks abgespeichert ist. Ein optionaler Drucksensor 31 misst ein Maß für den tatsächlichen Druck im Inneren der Druckkammer 16. Das Steuergerät empfängt Messwerte von dem Drucksensor 31 und steuert das Ventil 38 und die Fluidfördereinheit 30 abhängig von dem gemessenen aktuellen Druck in der Druckkammer 16 an, sodass der gemessene tatsächliche Druck dem vorgegebenen Verlauf folgt. In einer anderen Ausgestaltung steuert das Steuergerät den Druck gemäß dem vorgegebenen zeitlichen Verlauf, ohne dass notwendigerweise ein Drucksensor verwendet wird.

Bei dieser Regelung (Closed-loop Control) oder Steuerung (Open-loop Control) wird der tatsächliche Druck in der Druckkammer 16 abgesenkt, beispielsweise schrittweise oder auch kontinuierlich abgesenkt. Der vorgegebene zeitliche Verlauf des Solldrucks ist beispielsweise durch eine sogenannte Auftauchtabelle festgelegt und hängt von dem Überdruck ab, dem der Mensch P ausgesetzt war.

Jede Koppelstation 40.1 bis 40.4 für eine Atemstation umfasst jeweils
- ein Ventil 2.1 bis 2.4 in oder an der Inspirations-Leitung 1 und
- eine kammerseitige Schnellkupplung 10.1 bis 10.4 an der Exspirations-Leitung 11.

In Figur 1 wird beispielhaft eine erfindungsgemäße mobile Atemstation 50 gezeigt, die lösbar mit der ersten Koppelstation 40.1 verbunden ist. Der Mensch P nutzt die Atemstation 50, um sein Atemsystem und sein durchblutetes Gewebe zu dekomprimieren. Möglich ist, dass ein weiterer Mensch (nicht gezeigt) in der Dekompressionskammer 100 mindestens eine weitere erfindungsgemäße Atemstation (ebenfalls nicht gezeigt) zum Dekomprimieren verwendet, wobei diese weitere Atemstation mit einer weiteren Koppelstation 40.2 bis 40.4 lösbar verbunden ist.

Die erste Koppelstation 40.1 umfasst das Ventil 2.1 und die kammerseitige Schnellkupplung 10.1. Die Bauteile 2.1 und 10.1 fungieren als die beiden korrespondierenden kammerseitigen Koppeleinheiten.

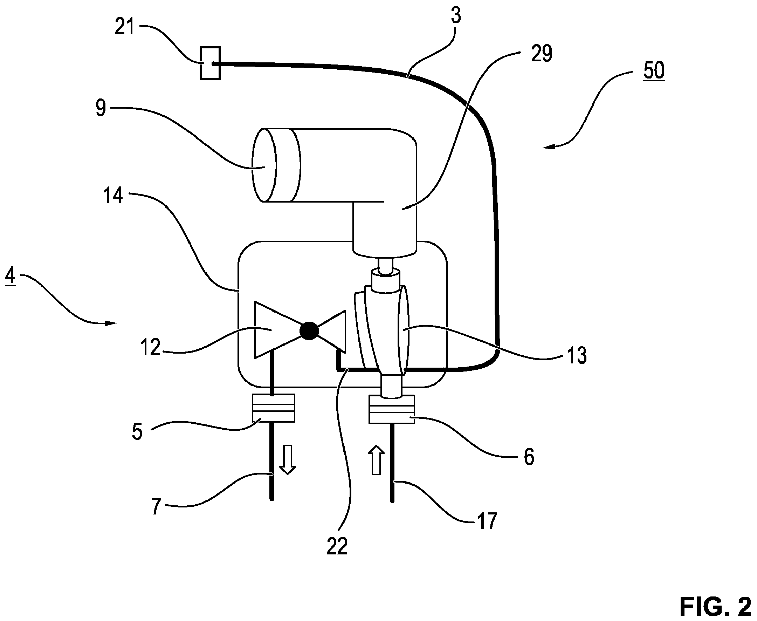

Figur 2 zeigt schematisch einen Teil der erfindungsgemäßen Atemstation 50.

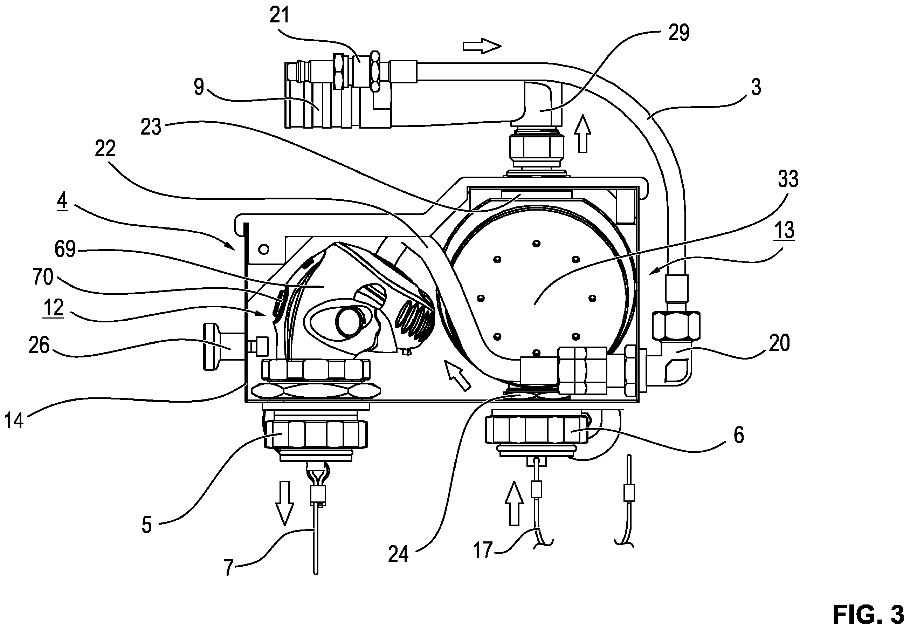

Figur 3 zeigt die Atemstation 50 in einer Seitenansicht. Gleiche Bezugszeichen haben die gleiche Bedeutung wie in Figur 1.

Die Atemstation 50 ist mobil, d.h. sie kann von einem Menschen P transportiert werden. Daher kann ein Mensch P die Atemstation 50 nacheinander in verschiedenen Dekompressionskammern oder an unterschiedlichen Koppelstationen, also an unterschiedlichen Paaren 2.1, 10.1, ..., 2.4, 10.4 von korrespondierenden Koppeleinheiten, in derselben Dekompressionskammer 100 benutzen.

Möglich ist, dass die Atemstation 50 einem Menschen P zugeordnet ist und nur von diesem Menschen P benutzt wird. Dies reduziert den Aufwand, der für ein Desinfizieren erforderlich ist, verglichen mit einer Atemstation 50, die nacheinander von verschiedenen Menschen benutzt wird. Außerdem kann die Atemstation 50 an einen bestimmten Menschen P angepasst sein, insbesondere an Abmessungen und respiratorische Parameter dieses Menschen P.

Die mobile Atemstation 50 des Ausführungsbeispiels umfasst
- eine atemstationsseitige Inspirations-Schnellkupplung 21, die als die Inspirations-Koppeleinheit fungiert,
- einen kammerseitigen Inspirations-Schlauch 3,
- eine Atemstelle 4 mit einem starren und druckfesten oder elastischen Gehäuse 14,
- einen pilzförmigen Haken 26 außen am Gehäuse 14,
- einen personenseitigen Inspirations-Schlauch 7, der als die Inspirations-Fluidführungseinheit fungiert,
- ein Rückschlagventil 18 im personenseitigen Inspirations-Schlauch 7,
- eine Atemmaske 8, welche wenigstens den Mund und die Nase der Person P bedeckt und als eine personenseitige Koppeleinheit fungiert,
- einen personenseitigen Exspirations-Schlauch 17, der als die Exspirations-Fluidführungseinheit fungiert,
- ein Rückschlagventil 19 im personenseitigen Exspirations-Schlauch 17,
- eine atemstationsseitige Exspirations-Schnellkupplung 9, die als die Exspirations-Koppeleinheit fungiert, und
- ein Verbindungsstück 29.

Der kammerseitige Inspirations-Schlauch 3 ist an einem Ende mit der atemstationsseitigen Inspirations-Schnellkupplung 21 fest und fluiddicht verbunden. Die atemstationsseitige Inspirations-Schnellkupplung 21 lässt sich lösbar und fluiddicht mit einem Ventil 2.1 bis 2.4 2.4 einer Koppelstation 40.1 bis 40.4 verbinden und wieder lösen. Die atemstationsseitige Exspirations-Schnellkupplung 9 lässt sich lösbar und fluiddicht mit einer kammerseitigen Schnellkupplung 10.1 bis 10.4 dieser Koppelstation 40.1 bis 40.4 verbinden und wieder lösen. Weil diese beiden Verbindungen lösbar sind, lässt die Atemstation 50 sich wahlweise mit der ersten Koppelstation 40.1 oder mit einer weiteren Koppelstation 40.2 bis 40.4 in der Druckkammer 16 verbinden und auch mit einer Koppelstation in einer anderen Dekompressionskammer (nicht gezeigt). Bevorzugt lassen diese beiden Verbindungen sich ohne Verwendung eines Werkzeugs herstellen und wieder lösen. Vielmehr kann ein Mensch P lediglich mit seinen Händen die Verbindungen herstellen und wieder lösen.

Das Verbindungsstück 29 verbindet die atemstationsseitige Exspirations-Schnellkupplung 9 mit dem Gehäuse 14, 14, welches bevorzugt starr und druckfest ist. Der kammerseitige Inspirations-Schlauch 3 verbindet die atemstationsseitige Inspirations-Schnellkupplung 21 fest und fluiddicht mit dem Gehäuse 14 der Atemstelle 4. Das Gehäuse 14 ist weiterhin fest und fluiddicht mit einer Inspirations-Schraubkupplung 5 und mit einer Exspirations-Schraubkupplung 6 verbunden. Der personenseitige Inspirations-Schlauch 7 ist fest und fluiddicht mit der Inspirations-Schraubkupplung 5 verbunden, der personenseitige Exspirations-Schlauch 17 fest und fluiddicht mit der Exspirations-Schraubkupplung 6. Beide Schläuche 7, 17 sind fluiddicht mit der Atemmaske 8 verbunden. Im Ausführungsbeispiel steht das Gehäuse 14 in einer Fluidverbindung mit der Umgebung, sodass der Druck im Inneren des Gehäuses 14 dem Druck im Inneren der Druckkammer 16 folgt. Bevorzugt ist es während eines normalen Einsatzes des Gehäuses 14 nicht erforderlich, das Gehäuse 14 zu öffnen.

Die Kennzeichnung "fluiddicht" schließt natürlich nicht aus, dass z.B. aufgrund von Ungenauigkeiten bei der Fertigung und von Temperaturunterschieden Spalten und Lecks auftreten. Eine "feste Verbindung" lässt sich mit mindestens einem geeigneten Werkzeug lösen, insbesondere zwecks einer Wartung oder Reinigung, aber in der Regel nicht ohne ein Werkzeug. Im Einsatz wird die feste Verbindung nicht gelöst.

Die Inspirations-Leitung 1 führt ein Gasgemisch von einer nicht gezeigten Quelle an den vier Ventilen 2.1 bis 2.4 vorbei. Jedes Ventil 2.1 bis 2.4 steht in jeweils einer Fluidverbindung mit der Inspirations-Leitung 1. Dieses Gasgemisch enthält Sauerstoff und wird im Folgenden als "Atemgas" bezeichnet. Der Anteil an Sauerstoff in dem Atemgas kann gleich dem oder höher als der Anteil von Sauerstoff in der Luft sein.

Die Atemmaske 8 lässt sich an den pilzförmigen Haken 26 hängen und steht dadurch griffbereit zur Verfügung. Optional hält ein Schnappverschluss die Atemmaske 8 lösbar am Haken 26.

Falls die Atemstation 50 mit der Koppelstation 40.1 verbunden und das Ventil 2.1 geöffnet ist, so ist eine Inspirations-Fluidverbindung zwischen der Inspirations-Leitung 1 und der Atemmaske 8 hergestellt. Das Atemgas kann dann auf folgendem Wege von der Quelle zur Atemmaske 8 fließen:
- durch die Inspirations-Leitung 1,
- durch das Ventil 2.1,
- durch die atemstationsseitige Inspirations-Schnellkupplung 21,
- durch den kammerseitigen Inspirations-Schlauch 3,
- durch die Atemstelle 4,
- durch die Inspirations-Schraubkupplung 5 und
- durch den personenseitigen Inspirations-Schlauch 7.

Die Atemluft, welche der Mensch P ausatmet, kann auf folgendem Wege von der Atemmaske 8 aus der Druckkammer 16 hinaus fließen:
- durch den personenseitigen Exspirations-Schlauch 17,
- durch die Exspirations-Schraubkupplung 6,
- durch die Atemstelle 4,
- durch das Verbindungsstück 29,
- durch die atemstationsseitige Exspirations-Schnellkupplung 9,
- durch die kammerseitige Schnellkupplung 10.1 und
- durch die Exspirations-Leitung 11.

Der kammerseitige Inspirations-Schlauch 3 der Atemstation 50 ist über eine Schraubkupplung 20 mit einem atemstellenseitigen Inspirations-Schlauch 22 verbunden. Die Schraubkupplung 20 ist in das Gehäuse 14 eingelassen.

Im Ausführungsbeispiel sind in dem Gehäuse 14 der Atemstelle 4
- ein Inspirations-Lungenautomat 12,
- ein Exspirations-Lungenautomat 13,
- der atemstellenseitige Inspirations-Schlauch 22 und
- zwei Röhren 23 und 24
angeordnet. Der atemstellenseitige Inspirations-Schlauch 22 verbindet den Inspirations-Lungenautomaten 12 fest und fluiddicht mit der Schraubkupplung 20. Die Röhre 24 verbindet die Exspirations-Schraubkupplung 6 fest und fluiddicht mit dem Exspirations-Lungenautomaten 13. Die Röhre 23 verbindet den Exspirations-Lungenautomaten 13 fest und fluiddicht mit der atemstationsseitigen Exspirations-Schnellkupplung 9.

Die Bauteile 20, 22, 12, 5 gehören zu der Inspirations-Fluidverbindung des Ausführungsbeispiels. Die Bauteile 6, 24, 13, 23 gehören zu der Exspirations-Fluidverbindung des Ausführungsbeispiels.

Figur 4 zeigt schematisch die beiden lösbaren Verbindungen zwischen der Atemstation 50 und der ersten Koppelstation 40.1. Gleiche Bezugszeichen haben wiederum die gleiche Bedeutung wie in Figur 1 bis Figur 3.

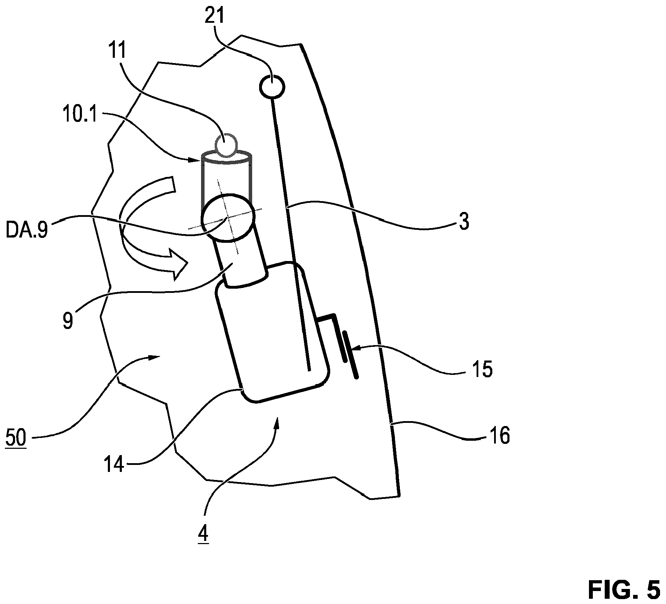

Figur 5 zeigt schematisch und beispielhaft, wie die Atemstation 50 sich lösbar an der Innenwand der Druckkammer 16 befestigen lässt. Diese Innenwand ist bevorzugt aus einem metallischen und daher magnetischen Werkstoff hergestellt. Außen am Gehäuse 14 der Atemstelle 4 ist ein Magnet 15 befestigt.

Dieser Magnet 15 ist bevorzugt ein Permanentmagnet und erfordert keine elektrische Energie, kann aber auch ein Elektromagnet sein, und hält die Atemstelle 4 lösbar an der Druckkammer 16. Außerdem trägt die starre Exspirations-Leitung 11 über die beiden Schnellkupplungen 10.1 und 9 die Atemstelle 4. Die atemstationsseitige Exspirations-Schnellkupplung 9 ist relativ zur kammerseitigen Schnellkupplung 10.1 um eine Drehachse DA.9 drehbar, die senkrecht auf der Zeichenebene von Figur 5 steht. Dadurch lässt sich zunächst die atemstationsseitige Exspirations-Schnellkupplung 9 mit der kammerseitigen Schnellkupplung 10.1 verbinden, und dann hält der Magnet 15 die Atemstation 50 lösbar an der Innenwand des Gehäuses 16.

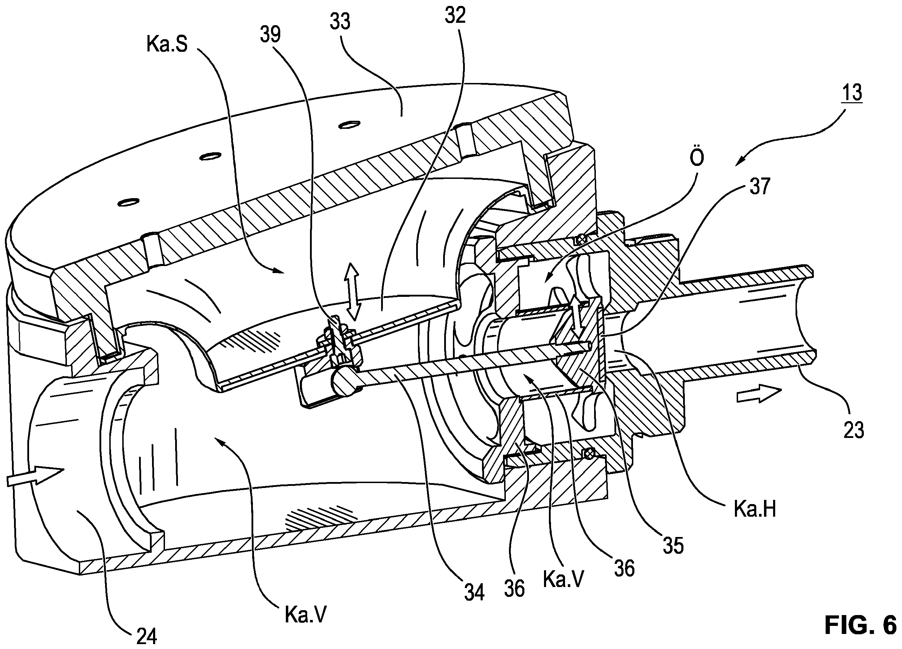

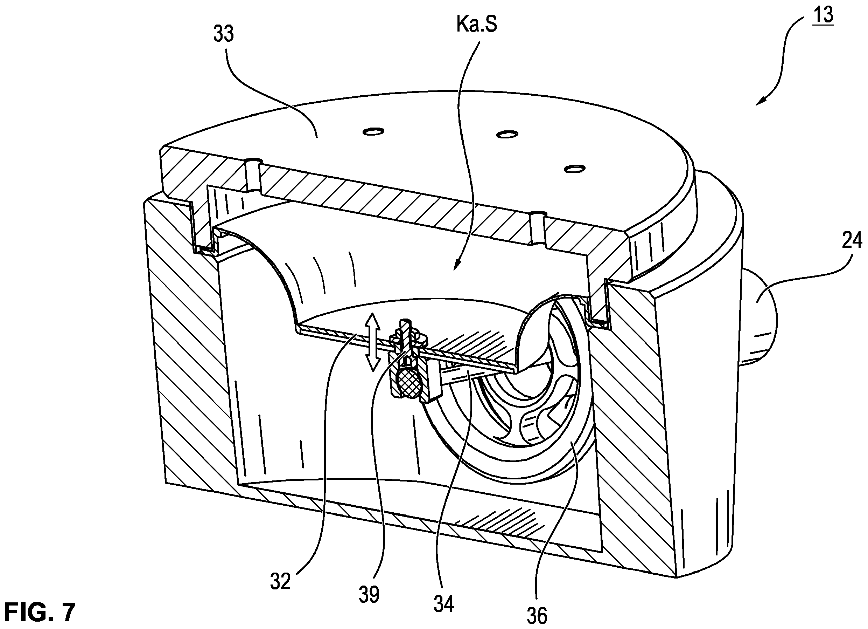

Figur 6 bis Figur 8 zeigen beispielhaft den Exspirations-Lungenautomaten 13 aus drei verschiedenen Betrachtungsrichtungen. Gezeigt werden folgende Bestandteile:
- ein flexibles Trennelement in Form einer Membrane 32,
- eine scheibenförmige Wand 33, die zum Gehäuse des Exspirations-Lungenautomaten 13 gehört,
- ein beweglicher Ventilkörper mit einer Dichtung 37 und einem Träger 35 für die Dichtung 37,
- eine Röhren-Halterung 27, die mit der Röhre 23 ein einziges Bauteil bildet,
- ein Ventilkörper-Sitz 36, welcher sich in der Röhren-Halterung 27 fortsetzt,
- ein Hebel 34 und
- ein mechanisches Verbindungselement 39.

In dem starren Gehäuse des Exspirations-Lungenautomaten 13 sind
- eine Vordruck-Kammer Ka.V,
- eine Hinterdruck-Kammer Ka.H und
- eine Steuerdruck-Kammer Ka.S
ausgebildet.

Der Inspirations-Lungenautomat 12 kann entsprechend aufgebaut sein und insbesondere ebenfalls drei Kammern umfassen.

Ein Teil der Vordruck-Kammer Ka.V befindet sich im Inneren der Röhre 36. Die Vordruck-Kammer Ka.V steht in einer Fluidverbindung mit der Röhre 24 und daher über den personenseitigen Exspirations-Schlauch 17 und die Exspirations-Schraubkupplung 6 in einer Fluidverbindung mit der Atemmaske 8. Deshalb steht die Vordruck-Kammer Ka.V in einer Fluidverbindung mit der Lunge des verbundenen Menschen P. Die Hinterdruck-Kammer Ka.H steht in einer Fluidverbindung mit der Röhre 23 und daher über die atemstationsseitige Exspirations-Schnellkupplung 9 und die kammerseitige Schnellkupplung 10.1 in einer Fluidverbindung mit der Exspirations-Leitung 11 und deshalb mit der Umgebung der Dekompressionskammer 100. Die Steuerdruck-Kammer Ka.S grenzt an die scheibenförmige Wand 33 an. In die Wand 33 sind Öffnungen eingelassen. Daher steht die Steuerdruck-Kammer Ka.S in einer Fluidverbindung mit dem Rest der Dekompressionskammer 100, also mit dem Inneren der Druckkammer 16.

Die flexible Membrane 32 trennt die Steuerdruck-Kammer Ka.S von der Vordruck-Kammer Ka.V. Weil die Membrane 32 flexibel ist, stellt sich ein Druckgleichgewicht zwischen dem Steuerdruck, das ist der Druck in der Steuerdruck-Kammer Ka.S, und dem Vordruck, das ist der Druck in der Vordruck-Kammer Ka.V, ein. Der Vordruck und der Steuerdruck wirken von zwei Seiten auf die Membrane 32 ein und legen daher die Position der Membrane 32 fest. Der Steuerdruck folgt dank der Öffnungen in der Wand 33 dem geregelten oder gesteuerten Druck in der Dekompressionskammer 100.

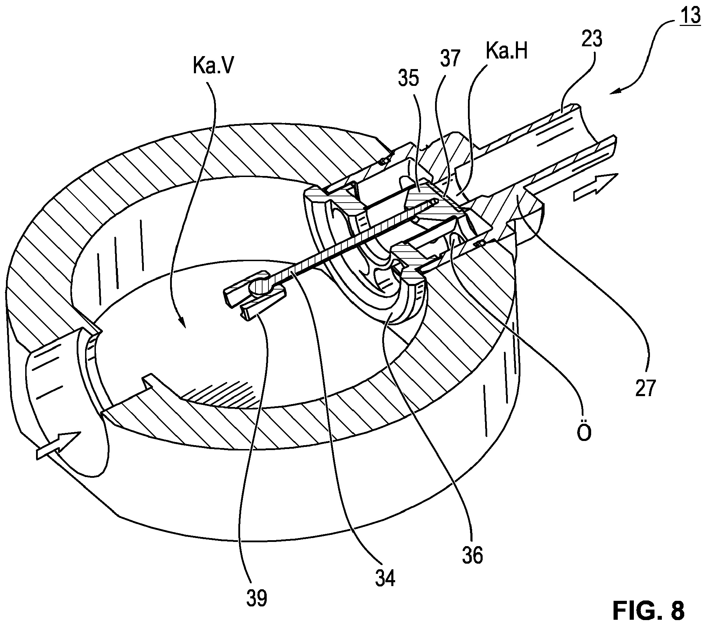

Die Vordruck-Kammer Ka.V steht in einer Fluidverbindung mit der Hinterdruck-Kammer Ka.H. Ein Ventilkörper mit einer Dichtung 37 und einem Träger 35 für die Dichtung 37 vermag diese Fluidverbindung wahlweise freizugeben oder zu verschließen. In den Träger 35 ist eine Öffnung Ö eingelassen. In der gezeigten Realisierungsform kann sich der Ventilkörper 37, 35 relativ zu dem Ventilkörper-Sitz 36 und der Röhre 23 bewegen, und zwar durch eine Art Kippbewegung relativ zu der übereinstimmenden Mittelachse der beiden Bauteile 36 und 23. In der Endposition, die in Figur 6 und Figur 8 gezeigt wird, verschließt die Dichtung 37 die Fluidverbindung zwischen der Vordruck-Kammer Ka.V und der Hinterdruck-Kammer Ka.H. Daher kann kein Gas aus dem Ventilkörper-Sitz 36 in die Röhre 23 fließen. In der anderen Endposition (nicht gezeigt) tritt ein Abstand zwischen der Dichtung 37 und der Hinterdruck-Kammer Ka.H auf. Dadurch wird ein Spalt gebildet. Die Öffnung Ö befindet sich zwischen der Vordruck-Kammer Ka.V und der Hinterdruck-Kammer Ka.H. Falls die Dichtung 37 die Fluidverbindung nicht verschließt, kann ein Gas aus der Vordruck-Kammer Ka.V durch die Öffnung Ö und den Spalt hindurch in die Hinterdruck-Kammer Ka.H fließen.

Der Ventilkörper 37, 35 steht in einer mechanischen Wirkverbindung mit der Membrane 32. Die mechanische Wirkverbindung koppelt den Ventilkörper 37, 35 mit der Membrane 32. Zu dieser mechanischen Wirkverbindung gehören das Verbindungselement 39 und der Hebel 34. Das Verbindungselement 39 verbindet das freie Ende des Hebels 34 mit der Membrane 32. Das andere Ende des Hebels 34 ist mit dem Ventilkörper 37, 35 verbunden. Der Hebel 34 vermag den Ventilkörper 37, 35 zu bewegen. In der gezeigten Realisierungsform vermag der Hebel 34 den Ventilkörper 37, 35 in zwei Richtungen senkrecht zu der übereinstimmenden Mittelachse der beiden Bauteile 36 und 23 zu verschwenken.

Die mechanische Wirkverbindung 39, 34 überträgt eine Bewegung der Membrane 32 auf den Ventilkörper 37, 35. Falls der Vordruck größer als der Steuerdruck ist, wird Folgendes bewirkt: Die Membrane 32 wird auf die Wand 33 zu bewegt, und die mechanische Wirkverbindung 39, 34 bewegt den Ventilkörper 37, 35 in die freigebende Position. Zwischen der Vordruck-Kammer Ka.V und der Hinterdruck-Kammer Ka.H findet ein Druckausgleich statt. Falls der Steuerdruck größer als der Vordruck ist, wird die Membrane 32 von der Wand 33 weg bewegt, und die mechanische Wirkverbindung 39, 34 bewegt den Ventilkörper 37, 35 in die verschließende Position.

Weil der Steuerdruck mit einem Zeitverzug dem Druck in der Umgebung der Atemstation 50 folgt, ist die Fluidverbindung zwischen der Vordruck-Kammer Ka.V und der Hinterdruck-Kammer Ka.H dann und nur dann hergestellt, wenn der Vordruck größer als der Umgebungsdruck ist. In der Regel ist der Vordruck daher stets kleiner oder gleich dem Umgebungsdruck.

Der Hebel 34 realisiert einen großen Hebelarm und einen kleinen Hebelarm. Dadurch bewirkt eine Bewegung der Membrane 32 über eine große Strecke, dass der Ventilkörper 37, 35 über eine kleine Strecke bewegt wird. Dank dieser beiden Hebelarme vermag die Membrane 32 den Ventilkörper 37, 35 zu bewegen, auch wenn von beiden Seiten am Ventilkörper 37, 35 erheblich unterschiedliche Drücke anliegen. Ein zusätzlicher Stellantrieb ist nicht erforderlich.

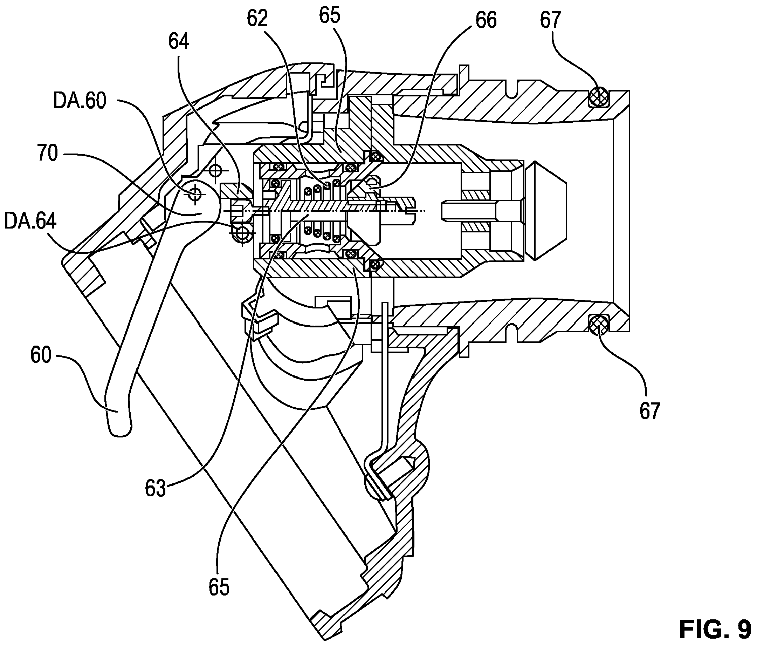

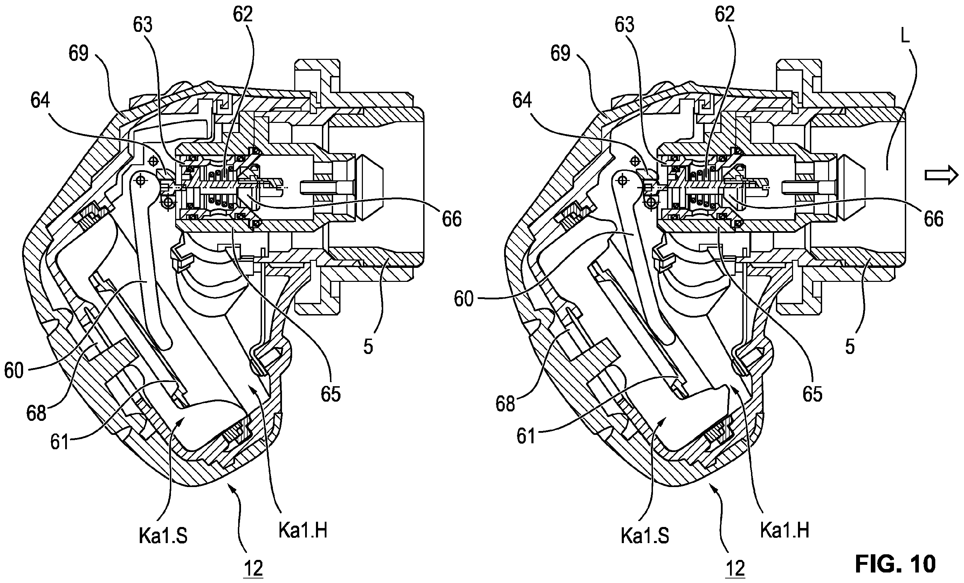

Figur 9 zeigt einen Bereich des Inspirations-Lungenautomaten 12 in einer Detailansicht, Figur 10 in einer Gesamtansicht. In einem Gehäuse 69 sind folgende weitere Bestandteile des Inspirations-Lungenautomaten 12 angeordnet:
- ein Ventilkörper 63,
- ein Ventilkörper-Sitz 65,
- ein Dichtkegel 66,
- eine Druckfeder 62,
- ein Haupthebel 60,
- ein sekundärer Hebel 64, der um eine Drehachse DA.64 drehbar ist,
- eine flexible und daher bewegliche Membrane 61,
- ein beweglicher Bereich 68 des Gehäuses 69 mit einem Stutzen und
- ein Dichtring 67.

Der Ventilkörper-Sitz 65 ist fest mit dem Gehäuse 69 verbunden. Der Ventilkörper 62 ist fest mit dem Dichtkegel 66 verbunden und relativ zu dem Ventilkörper-Sitz 65 linear zwischen einer verschließenden Position, die in Figur 9 gezeigt wird, und einer freigebenden Position beweglich. Die Druckfeder 62 stützt sich am Ventilkörper-Sitz 65 ab und ist bestrebt, den Ventilkörper 63 und damit den Dichtkegel 66 in einer verschließenden Position zu halten. In der verschließenden Position liegt der Dichtkegel 66 fest am Ventilkörper-Sitz 65 an. In der freigebenden Position tritt ein Abstand zwischen dem Dichtkegel 66 und dem Ventilkörper-Sitz 65 auf.

Der Haupthebel 60 ist um die Drehachse DA.60 drehbar und steht in einer mechanischen Wirkverbindung mit dem sekundären Hebel 64. Dank der Position der Drehachse DA.60 bildet der Haupthebel 60 einen langen Hebelarm und einen kurzen Hebelarm aus. Zum kurzen Hebelarm gehört ein Nocken 70, der an dem sekundären Hebel 64 anliegt. Die Druckfeder 62 drückt den sekundären Hebel 64 gegen den Haupthebel 60, wodurch der Haupthebel 66 gedreht wird, und zwar in Figur 9 gegen den Uhrzeigersinn. Wenn der Haupthebel 60 gegen die Kraft der Druckfeder 62 gedreht wird, und zwar in Figur 9 im Uhrzeigersinn, so drückt der Nocken 70 des Haupthebels 60 gegen den sekundären Hebel 64. Daraufhin wird der sekundäre Hebel 64 um die Achse DA.64 gedreht und drückt den Ventilkörper 63 und damit den Dichtkegel 66 gegen die Kraft der Druckfeder 62 aus der verschließenden Position in die freigebende Position.

Figur 10 zeigt links den Inspirations-Lungenautomaten 12 mit dem Dichtkegel 66 in der verschließenden Position und rechts mit dem Dichtkegel 66 in der freigebenden Position. In der freigebenden Position kann Atemluft L aus dem Inspirations-Lungenautomaten 12 in den personenseitigen Inspirations-Schlauch 7 fließen. Dank der Druckfeder 62 liegt der lange Hebelarm des Haupthebels 60 in beiden Positionen an der Membrane 61 an. Zwischen der Membrane 61 und dem beweglichen Bereich 68 des Gehäuses 69 wird eine Steuerdruck-Kammer Ka1.S gebildet. Die Membrane 61 trennt die Steuerdruck-Kammer Ka1.S fluiddicht von einer Hinterdruck-Kammer Ka1.H, in der sich der Haupthebel 60 befindet. Weil der Bereich 68 beweglich ist, folgt der Druck in der Steuerdruck-Kammer Ka1.S dem Druck im Inneren des Gehäuses 16.

Die Hinterdruck-Kammer Ka1.H steht in einer Fluidverbindung mit dem personenseitigen Inspirations-Schlauch 7. Wenn der Mensch P einatmet, so sinkt daher der Druck in der Hinterdruck-Kammer Ka1.H. Die Membrane 61 wird dadurch und durch den Druck in der Steuerdruck-Kammer Ka1.S gegen den Haupthebel 60 gedrückt. Dank des großen und des kleinen Hebelarms wird der Ventilkörper 63 gegen die Kraft der Druckfeder 62 verschoben und verschiebt den Dichtkegel 66 aus der verschließenden in die freigebende Position. Dadurch ist eine Fluidverbindung zwischen dem atemstellenseitigen Inspirations-Schlauch 22 und dem personenseitigen Inspirations-Schlauch 7 freigegeben.

Wenn der Mensch P aufhört einzuatmen und anschließend ausatmet, so steigt der Druck in der Hinterdruck-Kammer Ka1.H. Der höhere Druck in der Hinterdruck-Kammer Ka1.H bewegt die Membrane 61 auf den beweglichen Bereich 68 zu. Dadurch und dank der Federkraft der Druckfeder 62 wird der Ventilkörper 63 wieder verschoben. Der Dichtkegel 66 wird wieder in die verschließende Position bewegt. Die Fluidverbindung zwischen dem atemstellenseitigen Inspirations-Schlauch 22 ist unterbrochen.

Wie bereits dargelegt, wird der tatsächliche Druck im Inneren der Dekompressionskammer 100 durch eine Regelung oder Steuerung abgesenkt, beispielsweise schrittweise abgesenkt. Die Inspirations-Leitung 1 stellt das Atemgas, welches der Mensch P einatmet, mit einem Druck bereit, der bevorzugt größer ist als der größte Druck im Inneren der Dekompressionskammer 100. Dank des Inspirations-Lungenautomaten 12 ist es nicht erforderlich, den Druck in der Inspirations-Leitung 1 an den zeitlich veränderlichen vorgegebenen Solldruck anzupassen. Vielmehr folgt der Druck in der Hinterdruck-Kammer Ka1.H des Inspirations-Lungenautomaten 12 dem Steuerdruck und damit dem Druck im Inneren der Dekompressionskammer 100. Der Exspirations-Lungenautomat 13 stellt sicher, dass der Druck, mit dem der Mensch P ausatmet, ebenfalls dem geregelten oder gesteuerten Druck im Inneren der Dekompressionskammer 100 folgt.

**Bezugszeichenliste**

| | |
|---|---|
| 1 | Inspirations-Leitung, leitet Atemgas in die Dekompressionskammer 100 |
| 2.1, ..., 2.4 | Ventil in der Inspirations-Leitung 1, gehört zu der Koppelstation 40.1, ... , 40.4 |
| 3 | kammerseitiger Inspirations-Schlauch, verbindet die atemstationsseitige Inspirations-Schnellkupplung 21 mit der Atemstelle 4 |
| 4 | Atemstelle, gehört zur Atemstation 50, umfasst das Gehäuse 14, die Schraubkupplungen 5 und 6, den Inspirations-Lungenautomaten 12, den Exspirations-Lungenautomaten 13 und den atemstellenseitigen Inspirations-Schlauch 22 |
| 5 | Inspirations-Schraubkupplung am Gehäuse 14, mit dem personenseitigen Inspirations-Schlauch 7 verbunden, gehört zur Inspirations-Fluidverbindung |
| 6 | Exspirations-Schraubkupplung am Gehäuse 14, mit dem personenseitigen Exspirations-Schlauch 17 verbunden, gehört zur Exspirations-Fluidverbindung |
| 7 | personenseitiger Inspirations-Schlauch, verbindet die Atemstelle 4 mit der Atemmaske 8, fungiert als Inspirations-Fluidführungseinheit |
| 8 | Atemmaske für den Menschen P, mit den Schläuchen 7 und 17 verbunden, fungiert als personenseitige Koppeleinheit |
| 9 | stationsseitige Exspirations-Schnellkupplung, lässt sich lösbar mit einer kammerseitigen Schnellkupplung 10.1, ..., 10.4 verbinden, bildet zusammen mit dem Verbindungsstück 29 ein Winkelstück, fungiert als Exspirations-Koppeleinheit |
| 10.1, ..., 10.4 | kammerseitige Schnellkupplung an der Exspirations-Leitung 11, gehört zu der Koppelstation 40.1, ..., 40.4 |
| 11 | Exspirations-Leitung, führt in die Umgebung der Dekompressionskammer 100 |
| 12 | Inspirations-Lungenautomat, mit dem atemstellenseitigen Inspirations-Schlauch 22 und der Inspirations-Schraubkupplung 5 verbunden, umfasst die Hinterdruck-Kammer Ka1.H und die Steuerdruck-Kammer Ka1.S |
| 13 | Exspirations-Lungenautomat, mit der Exspirations-Schraubkupplung 6 und der atemstationsseitigen Exspirations-Schnellkupplung 9 verbunden, umfasst die Hinterdruck-Kammer Ka.H, die Steuerdruck-Kammer Ka.S und die Vordruck-Kammer Ka.V |
| 14 | Gehäuse der Atemstelle 4, trägt die Schraubkupplungen 5 und 6 sowie den Haken 26 |
| 15 | Magnet, mit dem Gehäuse 14 der Atemstelle 4 verbunden oder verbindbar |
| 16 | Druckkammer der Dekompressionskammer 100 |
| 17 | personenseitiger Exspirations-Schlauch, verbindet die Atemmaske 8 mit der Atemstelle 4, fungiert als Exspirations-Fluidführungseinheit |
| 18 | Rückschlagventil im personenseitigen Inspirations-Schlauch 7 |
| 19 | Rückschlagventil im personenseitigen Exspirations-Schlauch 17 |
| 20 | Schraubkupplung am Gehäuse 14, außen mit dem kammerseitigen Inspirations-Schlauch 3 und innen mit dem atemstellenseitigen Inspirations-Schlauch 22 verbunden, gehört zur Inspirations-Fluidverbindung |
| 21 | atemstationsseitige Inspirations-Schnellkupplung, mit dem kammerseitigen Inspirations-Schlauch 3 verbunden, lässt sich lösbar mit einem Ventil 2.1, ..., 2.4 verbinden, fungiert als Inspirations-Koppeleinheit |
| 22 | atemstellenseitiger Inspirations-Schlauch, verbindet die Schraubkupplung 20 mit dem Inspirations-Lungenautomat 12, gehört zur Inspirations-Fluidverbindung |
| 23 | Röhre in der Atemstelle 4, verbindet den Exspirations-Lungenautomaten 13 über das Verbindungsstück 29 mit der atemstationsseitige Exspirations-Schnellkupplung 9, gehört zur Exspirations-Fluidverbindung |
| 24 | Röhre in der Atemstelle 4, verbindet die Exspirations-Schraubkupplung 6 mit dem Exspirations-Lungenautomaten 13, gehört zur Exspirations-Fluidverbindung |
| 26 | pilzförmiger Haken außen am Gehäuse 14, vermag die Atemmaske 8 zu tragen |
| 27 | Röhren-Halterung für die Röhre 23, mit dem Ventilkörper-Sitz 36 verbunden |
| 29 | Verbindungsstück zwischen der Exspirations-Schnellkupplung 9 und der Röhre 23, bildet zusammen mit der stationsseitigen Exspirations-Schnellkupplung 9 ein Winkelstück |
| 30 | ansteuerbare Fluidfördereinheit, welche den Druck in der Dekompressionskammer 100 einstellt |
| 31 | optionaler Drucksensor, misst den Druck im Inneren der Druckkammer 16 |
| 32 | Membrane, fungiert als flexibles Trennelement des Exspirations-Lungenautomaten 13 |
| 33 | scheibenförmige Wand des Exspirations-Lungenautomaten 13, begrenzt die Steuerdruck-Kammer Ka.S, hat Öffnungen |
| 34 | Hebel, gehört zu einer mechanischen Wirkverbindung zwischen der Membrane 32 und dem Ventilkörper 37, 35 |
| 35 | Träger für die Dichtung 37, gehört zum Ventilkörper |
| 36 | Ventilkörper-Sitz für den Ventilkörper 37, 35 |
| 37 | Dichtung, gehört zum Ventilkörper |
| 38 | ansteuerbares Ventil, vermag Druck aus der Druckkammer 16 abzulassen |
| 39 | mechanisches Verbindungselement zwischen dem Hebel 34 und der Membrane 32 |
| 40.1, ... , 40.4 | Koppelstationen, umfassen jeweils ein Ventil 2.1, ..., 2.4 und eine kammerseitige Schnellkupplung 10.1, ..., 10.4 |
| 50 | Atemstation, aktuell mit der ersten Koppelstation 40.1 verbunden, umfasst die atemstationsseitige Inspirations-Schnellkupplung 21, die atemstationsseitige Exspirations-Schnellkupplung, das Verbindungsstück 29, den kammerseitiger Inspirations-Schlauch 3, die Atemstelle 4 mit den Lungenautomaten 12 und 13, die Schläuche 7 und 17 und die Atemmaske 8 |
| 60 | Haupthebel des Inspirations-Lungenautomaten 12, umfasst den Nocken 70 |
| 61 | Membrane des Inspirations-Lungenautomaten 12 |
| 62 | Druckfeder, ist bestrebt, den Ventilkörper 63 in eine sperrende Position zu bewegen |
| 63 | Ventilkörper, fest mit dem Dichtkegel 66 verbunden |
| 64 | sekundärer Hebel, überträgt eine Bewegung des Haupthebels 60 auf den Ventilkörper 63 |
| 65 | Ventilkörper-Sitz |
| 66 | Dichtkegel, fest mit dem Ventilkörper 63 verbunden |
| 67 | Dichtring |
| 68 | beweglicher Bereich des Gehäuses 69 mit Stutzen |
| 69 | Gehäuse des Inspirations-Lungenautomaten 12 |
| 70 | Nocken, gehört zum Haupthebel 60 |
| 100 | Dekompressionskammer, umfasst die Druckkammer 16, die Inspirations-Leitung 1, die Exspirations-Leitung 11, die vier Koppelstationen 40.1 bis 40.4 und den Drucksensor 31 |
| DA. 9 | Drehachse, um welche die atemstationsseitige Exspirations-Schnellkupplung 9 relativ zur kammerseitige Schnellkupplung 10.1 drehbar ist |
| DA.60 | Drehachse, um welche der Hebel 60 drehbar ist |
| Ka.H | Hinterdruck-Kammer des Exspirations-Lungenautomaten 13 |
| Ka.S | Steuerdruck-Kammer des Exspirations-Lungenautomaten 13 |
| Ka.V | Vordruck-Kammer des Exspirations-Lungenautomaten 13 |
| Ka1.H | Hinterdruck-Kammer des Inspirations-Lungenautomaten 12 |
| Ka1.S | Steuerdruck-Kammer des Inspirations-Lungenautomaten 12 |
| P | Mensch, der mit der Atemstation 50 verbunden ist und die Atemmaske 8 trägt, ist zu dekomprimieren |

## Patentansprüche

1. Mobile Atemstation (50) zur Verwendung in einer Dekompressionskammer (100) zum Dekomprimieren eines Menschen (P),
wobei die Atemstation (50)
- eine personenseitige Koppeleinheit (8), die mit dem Atemsystem eines Menschen (P) verbunden oder verbindbar ist,
- eine Inspirations-Koppeleinheit (21),
- eine Exspirations-Koppeleinheit (9),
- eine Inspirations-Fluidführungseinheit (7) und
- eine Exspirations-Fluidführungseinheit (17)
umfasst,
wobei die Inspirations-Koppeleinheit (21) und die Exspirations-Koppeleinheit (9) der Atemstation (50) mit jeweils einer korrespondierenden Koppeleinheit (2.1 bis 2.4, 10.1 bis 10.4) einer Koppelstation (40.1 bis 40.4) einer Dekompressionskammer (100) lösbar verbindbar sind,
wobei die Inspirations-Koppeleinheit (21) in einer Inspirations-Fluidverbindung (20, 22, 12, 5) mit der Inspirations-Fluidführungseinheit (7) steht,
wobei die Exspirations-Fluidführungseinheit (17) in einer Exspirations-Fluidverbindung (6, 24, 13, 23) mit der Exspirations-Koppeleinheit (9) steht und
wobei die Atemstation (50) dazu ausgestaltet ist,
- ein Gasgemisch umfassend Sauerstoff von der Inspirations-Koppeleinheit (21) durch die Inspirations-Fluidverbindung (20, 22, 12, 5) und die Inspirations-Fluidführungseinheit (7) hindurch zur personenseitigen Koppeleinheit (8) zu leiten und
- ein Gasgemisch, das ein Mensch (P) in die personenseitige Koppeleinheit (8) hinein ausgeatmet hat, von der personenseitigen Koppeleinheit (8) durch die Exspirations-Fluidführungseinheit (17) und die Exspirations-Fluidverbindung (6, 24, 13, 23) hindurch zur Exspirations-Koppeleinheit (9) zu leiten.

2. Mobile Atemstation (50) nach Anspruch 1,
**dadurch gekennzeichnet, dass**
die Verbindungen zwischen den beiden Koppeleinheiten (21, 9) der Atemstation (50) und den beiden korrespondierenden Koppeleinheiten (2.1 bis 2.4, 10.1 bis 10.4) der Dekompressionskammer (100) sich ohne Verwendung eines Werkzeugs herstellen und wieder lösen lassen.

3. Mobile Atemstation (50) nach einem der vorhergehenden Ansprüche,
**dadurch gekennzeichnet, dass**
die Inspirations-Koppeleinheit (21, 9) und die Exspirations-Koppeleinheit (9) jeweils eine Schnellkupplung umfassen,
wobei jede Schnellkupplung einer Koppeleinheit (21, 9) der Atemstation (50) mit jeweils einer korrespondierenden Schnellkupplung einer korrespondierenden Koppeleinheit (2.1 bis 2.4, 10.1 bis 10.4) einer Dekompressionskammer (100) lösbar verbindbar ist.

4. Mobile Atemstation (50) nach einem der vorhergehenden Ansprüche,
**dadurch gekennzeichnet, dass**
die Atemstation (50) einen Inspirations-Lungenautomaten (12) umfasst,
wobei der Inspirations-Lungenautomat (12)
- in einer Fluidverbindung (20, 22) mit der Inspirations-Koppeleinheit (21) und in einer Fluidverbindung (5) mit der Inspirations-Fluidführungseinheit (7) steht und
- dazu ausgestaltet ist, den an der personenseitigen Koppeleinheit (8) anliegenden Druck so zu verändern, dass der anliegende Druck einem Druck in der Umgebung der Atemstation (50) folgt.

5. Mobile Atemstation (50) nach Anspruch 4,
**dadurch gekennzeichnet, dass**
der Inspirations-Lungenautomat (12)
- eine Vordruck-Kammer,
- eine Hinterdruck-Kammer und
- eine Steuerdruck-Kammer
umfasst,
wobei die Vordruck-Kammer in der Fluidverbindung (20, 22) mit der Inspirations-Koppeleinheit (21) steht,
wobei die Hinterdruck-Kammer in der Fluidverbindung (5) mit der Inspirations-Fluidführungseinheit (7) steht,
wobei die Steuerdruck-Kammer in einer Fluidverbindung mit der Umgebung der Atemstation (50) steht und
wobei der Inspirations-Lungenautomat (12) so ausgestaltet ist, dass zwischen der Vordruck-Kammer und der Hinterdruck-Kammer dann, bevorzugt nur dann, eine Fluidverbindung hergestellt ist,
wenn der Druck in der Steuerdruck-Kammer größer als der Druck in der Hinterdruck-Kammer ist.

6. Mobile Atemstation (50) nach einem der vorhergehenden Ansprüche,
**dadurch gekennzeichnet, dass**
die Atemstation (50) einen Exspirations-Lungenautomaten (13) umfasst, wobei der Exspirations-Lungenautomat (13)
- in einer Fluidverbindung (6, 24) mit der Exspirations-Fluidführungseinheit (17) und in einer Fluidverbindung (23, 29) mit der Exspirations-Koppeleinheit (9) steht und
- so ausgestaltet ist, dass dann und nur dann eine Fluidverbindung zwischen der Exspirations-Fluidführungseinheit (17) und der Exspirations-Koppeleinheit (9) durch den Exspirations-Lungenautomaten (13) hindurch hergestellt ist,
wenn der Druck in der Exspirations-Fluidführungseinheit (17) größer als der Druck in der Umgebung der Atemstation (50) ist.

7. Mobile Atemstation (50) nach Anspruch 6,
**dadurch gekennzeichnet, dass**
der Exspirations-Lungenautomat (13)
- eine Vordruck-Kammer (Ka.V),
- eine Hinterdruck-Kammer (Ka.H) und
- eine Steuerdruck-Kammer (Ka.S)
umfasst,
wobei die Vordruck-Kammer (Ka.V) in der Fluidverbindung (6, 24) mit der Exspirations-Fluidführungseinheit (17) steht,
wobei die Hinterdruck-Kammer (Ka.H) in der Fluidverbindung (23, 29) mit der Exspirations-Koppeleinheit (9) steht,
wobei die Steuerdruck-Kammer (Ka.S) in einer Fluidverbindung (33) mit der Umgebung der Atemstation (50) steht und
wobei der Exspirations-Lungenautomat (13) so ausgestaltet ist, dass zwischen der Vordruck-Kammer (Ka.V) und der Hinterdruck-Kammer (Ka.H) dann und nur dann eine Fluidverbindung hergestellt ist, wenn der Druck in der Steuerdruck-Kammer (Ka.S) kleiner als der Druck in der Vordruck-Kammer (Ka.V) ist.

8. Mobile Atemstation (50) nach einem der vorhergehenden Ansprüche,
**dadurch gekennzeichnet, dass**
die Atemstation (50) einen Inspirations-Lungenautomaten (12) und einen Exspirations-Lungenautomaten (13) umfasst,
wobei dann, wenn ein mit der personenseitigen Koppeleinheit (8) verbundener Mensch (P) einatmet,
- der Inspirations-Lungenautomat (12) die Inspirations-Fluidverbindung (20, 22, 12, 5) 5) 5) dergestalt freigibt, dass der an der personenseitigen Koppeleinheit (8) anliegende Druck einem Druck in der Umgebung der Atemstation (50) folgt und
- der Exspirations-Lungenautomat (13) die Exspirations-Fluidverbindung (6, 24, 13, 23) sperrt, und
wobei dann, wenn der verbundene Mensch (P) ausatmet,
- der Inspirations-Lungenautomat (12) die Inspirations-Fluidverbindung (20, 22, 12, 5) sperrt und
- der Exspirations-Lungenautomat (13) die Exspirations-Fluidverbindung (6, 24, 13, 23) dann freigibt, wenn der Druck in der Exspirations-Fluidführungseinheit (17) größer als der Druck in der Umgebung der Atemstation (50) ist, ist, und ansonsten versperrt.

9. Mobile Atemstation (50) nach einem der vorhergehenden Ansprüche,
**dadurch gekennzeichnet, dass**
die Atemstation (50) ein Gehäuse (14) umfasst,
wobei die Inspirations-Koppeleinheit (21) und die Exspirations-Koppeleinheit (9) außen am Gehäuse (14) befestigt sind,
wobei die personenseitige Koppeleinheit (8) außerhalb des Gehäuses (14) angeordnet ist und
wobei die Inspirations-Fluidführungseinheit (7) und die Exspirations-Fluidführungseinheit (17) das Gehäuse (14) mit der personenseitigen Koppeleinheit (8) verbinden.

10. Anordnung umfassend
- eine Dekompressionskammer (100) und
- mindestens eine erste mobile Atemstation (50),
wobei die Dekompressionskammer (100) zum Dekomprimieren eines Menschen (P) ausgestaltet ist und
- eine Druckkammer (16),
- eine Druckveränderungseinheit (30, 38),
- eine kammerseitige Inspirations-Fluidführungseinheit (1),
- eine kammerseitige Exspirations-Fluidführungseinheit (11) und
- eine erste Koppelstation (40.1)
umfasst,
wobei die erste mobile Atemstation (50) nach einem der vorhergehenden Ansprüche ausgestaltet ist,
wobei die erste Koppelstation (40.1)
- im Inneren der Druckkammer (16) angeordnet ist und
- eine kammerseitige Inspirations-Koppeleinheit (2.1) und eine kammerseitige Exspirations-Koppeleinheit (10.1) umfasst,
wobei die kammerseitige Inspirations-Koppeleinheit (2.1) der ersten Koppelstation (40.1) wenigstens zeitweise in einer Fluidverbindung mit der kammerseitigen Inspirations-Fluidführungseinheit (1) steht,
wobei die kammerseitige Exspirations-Koppeleinheit (10.1) der ersten Koppelstation (40.1) wenigstens zeitweise in einer Fluidverbindung mit der kammerseitigen Exspirations-Fluidführungseinheit (11) steht,
wobei die Inspirations-Koppeleinheit (21) der ersten Atemstation (50) mit der kammerseitigen Inspirations-Koppeleinheit (2.1) der ersten Koppelstation (40.1) wenigstens zeitweise lösbar verbunden oder verbindbar ist,
wobei die Exspirations-Koppeleinheit (9) der ersten Atemstation (50) mit der kammerseitigen Exspirations-Koppeleinheit (10.1) der ersten Koppelstation (40.1) wenigstens zeitweise lösbar verbunden oder verbindbar ist,
wobei die Druckveränderungseinheit (30, 38) dazu ausgestaltet ist, den Druck im Inneren der Druckkammer (16) dergestalt zu verändern, dass der Druck einem vorgegebenen zeitlichen Verlauf des Solldrucks folgt, und
wobei die Dekompressionskammer (100) dazu ausgestaltet ist, bei einer hergestellten Verbindung zwischen der ersten Atemstation (50) und der ersten Koppelstation (40.1)
- ein Gasgemisch umfassend Sauerstoff durch die kammerseitige Inspirations-Fluidführungseinheit (1), die kammerseitige Inspirations-Koppeleinheit (2.1) der ersten Koppelstation (40.1) und die erste Atemstation (50) hindurch zur personenseitigen Koppeleinheit (8) zu leiten und
- ein Gasgemisch, das der Mensch (P) in die personenseitige Koppeleinheit (8) hinein ausgeatmet hat, von der personenseitigen Koppeleinheit (8) durch die erste Atemstation (50), die kammerseitige Exspirations-Koppeleinheit (10.1) der ersten Koppelstation (40.1) und die kammerseitige Exspirations-Fluidführungseinheit (11) hindurch aus der Druckkammer (16) abzuführen.

11. Anordnung nach Anspruch 10,
**dadurch gekennzeichnet, dass**
die Dekompressionskammer (100) mindestens eine weitere Koppelstation (40.2 bis 40.4) umfasst,
wobei die oder jede weitere Koppelstation (40.2 bis 40.4)
- im Inneren der Druckkammer (16) angeordnet ist und
- jeweils eine weitere kammerseitige Inspirations-Koppeleinheit (2.2 bis 2.4) und eine weitere kammerseitige Exspirations-Koppeleinheit (10.2 bis 10.4) umfasst,
wobei die oder jede weitere kammerseitige Inspirations-Koppeleinheit (2.2 bis 2.4) wenigstens zeitweise in einer Fluidverbindung mit der kammerseitigen Inspirations-Fluidführungseinheit (1) steht,
wobei die oder jede weitere kammerseitige Exspirations-Koppeleinheit (10.2 bis 10.4) wenigstens zeitweise in einer Fluidverbindung mit der kammerseitigen Exspirations-Fluidführungseinheit (11) steht und
wobei die erste Atemstation (50) wahlweise mit der ersten Koppelstation (40.1) oder mit der oder einer weiteren Koppelstation (40.2) wenigstens zeitweise lösbar verbunden oder verbindbar ist.

12. Anordnung nach einem der Ansprüche 10 oder 11,
**dadurch gekennzeichnet, dass**
die Anordnung eine zweite mobile Atemstation nach einem der Ansprüche 1 bis 9 umfasst,
wobei wahlweise die erste Atemstation (50) oder die zweite Atemstation mit der ersten Koppelstation (40.1) lösbar verbunden oder verbindbar ist.

13. Anordnung nach einem der Ansprüche 10 bis 12,
**dadurch gekennzeichnet, dass** die Anordnung
- m Koppelstationen (40.1 bis 40.4) mit jeweils einer kammerseitigen Inspirations-Koppeleinheit (2.1 bis 2.4) und einer kammerseitigen Exspirations-Koppeleinheit (10.1 bis 10.4) und
- n mobile Atemstationen (50)
umfasst,
wobei m und n zwei natürliche Zahlen sind und n >= m > 1 gilt,
wobei die m Koppelstationen (40.1 bis 40.4) im Inneren der Druckkammer (16) angeordnet sind und
wobei jede der n mobilen Atemstationen (50)
- nach einem der Ansprüche 1 bis 9 ausgestaltet ist und
- sich mit jeweils einer der m Koppelstationen (40.1 bis 40.4) lösbar verbinden lässt.

14. Anordnung nach Anspruch 13,
**dadurch gekennzeichnet, dass**
sich mindestens eine der n Atemstationen im Inneren der Druckkammer (16) befindet,
ohne mit einer Koppelstation (40.1 bis 40.4) und ohne mit einem Menschen (P) verbunden zu sein,
vorbei bevorzugt n > m gilt.

15. Verfahren zum Betreiben einer Anordnung,
wobei die Anordnung
- eine Dekompressionskammer (100) mit m Koppelstationen (40.1 bis 40.4) und
- n mobile Atemstationen (50)
umfasst,
wobei m und n zwei natürliche Zahlen sind und n >= m > 1 gilt,
wobei die Dekompressionskammer (100) zum Dekomprimieren eines Menschen (P) ausgestaltet ist und
- eine Druckkammer (16),
- eine Druckveränderungseinheit (30, 38),
- eine kammerseitige Inspirations-Fluidführungseinheit (1) und
- eine kammerseitige Exspirations-Fluidführungseinheit (11) umfasst,
wobei jede Koppelstation (40.1 bis 40.4)
- im Inneren der Druckkammer (16) angeordnet ist und
- jeweils eine kammerseitige Inspirations-Koppeleinheit (2.1 bis 2.4) und eine kammerseitige Exspirations-Koppeleinheit (10.1 bis 10.4) umfasst,
wobei jede Atemstation (50) jeweils
- eine personenseitige Koppeleinheit (8), die mit dem Atemsystem eines Menschen (P) verbunden oder verbindbar ist,
- eine Inspirations-Koppeleinheit (21),
- eine Exspirations-Koppeleinheit (9),
- eine Inspirations-Fluidführungseinheit (7) und
- eine Exspirations-Fluidführungseinheit (17)
umfasst,
wobei die jeweilige Inspirations-Koppeleinheit (21) jeder Atemstation (50) mit jeweils zwei korrespondierenden Koppeleinheiten (2.1 bis 2.4) einer Koppelstation (40.1 bis 40.4) lösbar verbindbar sind,
wobei die jeweilige Exspirations-Koppeleinheit (9) jeder Atemstation (50) mit jeweils zwei korrespondierenden Koppeleinheiten (10.1 bis 10.4) einer Koppelstation (40.1 bis 40.4) lösbar verbindbar sind,
wobei die jeweilige Inspirations-Koppeleinheit (21) jeder Atemstation (50) in einer Inspirations-Fluidverbindung (20, 22, 12, 5) mit der jeweiligen Inspirations-Fluidführungseinheit (7) steht,
wobei die jeweilige Exspirations-Fluidführungseinheit (17) jeder Atemstation (50) in einer Exspirations-Fluidverbindung (6, 24, 13, 23) mit der jeweiligen Exspirations-Koppeleinheit (9) steht und
wobei das Verfahren die Schritte umfasst, dass
- in der Druckkammer (16) wenigstens eine Atemstation, bevorzugt mehrere Atemstationen, einer Menge von maximal m Atemstationen der n Atemstationen lösbar mit jeweils einer Koppelstation (40.1 bis 40.4) sowie mit jeweils einem Menschen (P) verbunden wird,
- ein Gasgemisch umfassend Sauerstoff mittels der kammerseitigen Inspirations-Fluidführungseinheit zu jeder Koppelstation (40.1 bis 40.4) geleitet wird und
- ein Gasgemisch, welches ein verbundener Mensch (P) ausgeatmet hat, mittels der kammerseitigen Explorations-Fluidführungseinheit von der jeweiligen Koppelstation (40.1 bis 40.4) abgeführt wird,
wobei die oder jede Atemstation, die mit einer Koppelstation (40.1 bis 40.4) verbunden ist,
- ein Gasgemisch umfassend Sauerstoff von der Inspirations-Koppeleinheit (21) durch die Inspirations-Fluidverbindung (20, 22, 12, 5) und die Inspirations-Fluidführungseinheit (7) hindurch zur personenseitigen Koppeleinheit (8) leitet und
- ein Gasgemisch, das ein Mensch (P) in die personenseitige Koppeleinheit (8) hinein ausgeatmet hat, von der personenseitigen Koppeleinheit (8) durch die Exspirations-Fluidführungseinheit (17) und die Exspirations-Fluidverbindung (6, 24, 13, 23) hindurch zu der Exspirations-Koppeleinheit (9) leitet.

16. Verfahren nach Anspruch 15,
**dadurch gekennzeichnet, dass**
n > m gilt und
n - m Atemstationen der Anordnung im Inneren der Druckkammer (16) vorrätig gehalten werden, ohne mit einer Koppelstation (40.1 bis 40.4) und ohne mit einem Menschen (P) verbunden zu werden und
mindestens einmal der Schritt durchgeführt wird, dass
eine Atemstation (50) aus der Menge der maximal m verbundenen Atemstationen durch eine Atemstation aus der Menge der n - m Atemstationen, die im Inneren der Druckkammer (16) vorrätig gehalten werden, ersetzt wird,
wobei bevorzugt der Schritt, die eine Atemstation durch die andere Atemstation zu ersetzen, den Schritt umfasst, dass
die eine Atemstation (50) von der Koppelstation (40.1 bis 40.4), mit der diese Atemstation verbunden war, gelöst wird und die andere Atemstation mit dieser Koppelstation verbunden wird.
